# EUROPEAN PATENT APPLICATION

(11) **EP 3 385 305 A1**
(43) Date of publication of application: **10.10.2018**
(21) Application number: 16870659.6
(22) Date of filing: 29.11.2016
(51) Int. Cl.: C08J 3/02, C07K 1/14, C07K 14/435, C08L 89/00

(54) **METHOD FOR PRODUCING PROTEIN SOLUTION**

(30) Priority: 01.12.2015 JP 2015235079
(71) Applicant: Spiber Inc., Yamagata 997-0052 (JP); Kojima Industries Corporation, Toyota-shi, Aichi 471-8588 (JP); Tekunohama Co., Ltd., Toyota-shi, Aichi 470-0441 (JP)
(72) Inventor: KOTAKA Koichi, Tsuruoka-shi Yamagata 997-0052 (JP); ISHII Takaoki, Tsuruoka-shi Yamagata 997-0052 (JP); SUGAHARA Junichi, Tsuruoka-shi Yamagata 997-0052 (JP); SATO Ryota, Tsuruoka-shi Yamagata 997-0052 (JP); NOBA Junichi, Toyota-shi Aichi 470-0441 (JP)
(74) Representative: Piotrowicz, Pawel Jan Andrzej
(86) International application number: PCT/JP2016/085409
(87) International publication number: WO 2017/094722

(57) **Abstract**

Provided is a method for producing a protein solution, including applying a pressure to a dispersion liquid containing a protein and a polar solvent in which the protein is dispersed, to obtain a protein solution containing the protein and the polar solvent in which the protein is dissolved.

## Description

### Technical Field

The present invention relates to a method for producing a protein solution. The present invention also relates to a method for producing a molded article using a protein solution.

### Background Art

As consciousness of environmental conservation increases, studies on alternative substances for petroleum-derived materials are progressing, and structural proteins. which are excellent in terms of strength, and the like are considered as a candidate. For example, molded articles such as cast films or fibers formed of structural proteins have been proposed.

Meanwhile, structural proteins are generally hydrophobic polymers, which are poorly soluble in polar solvents such as water. It is also conceivable to obtain a concentrated protein solution by heating and distilling off a solvent from a dilute solution of a structural protein. However, this operation may cause denaturation, gelation, degradation, and the like of the protein, resulting in a decrease in yield.

As a method for solving the above problem, Patent Literature 1 proposes a concentrating method for a protein aqueous solution, including bringing the protein aqueous solution into contact with an aqueous solution of a polyether-based polymer or polyol-based polymer via a dialysis membrane.

### Citation List

### Patent Literature

[Patent Literature 1] Japanese Unexamined Patent Publication No. H10-036676

### Summary of Invention

### Technical Problem

However, in a case of the concentrating method described in Patent Literature 1, it is necessary to separately provide an independent step such as a dialysis step after preparing the protein solution, and thus production steps become complicated. It is desirable to obtain a high-concentration protein solution in a simpler method.

Therefore, a main object of the present invention is to provide a method that makes it possible to easily obtain a high-concentration protein solution without requiring dialysis or the like.

### Solution to Problem

As a result of intensive studies to solve the above problem, the present inventors have found that by preparing a solution under pressure, it is possible to obtain a protein solution under milder conditions while suppressing alteration or the like of a protein. Based on this finding, the present inventors have completed the present invention.

According to an aspect of the present invention, there is provided a method for producing a protein solution, including: applying a pressure to a dispersion liquid containing a protein and a polar solvent in which the protein is dispersed, to obtain a protein solution containing the protein and the polar solvent in which the protein is dissolved.

With this method, it is possible to easily obtain a high-concentration protein solution without performing a step such as dialysis. For example, it is possible to obtain a protein solution containing a spider silk fibroin and a polar solvent in which the spider silk fibroin is dissolved, in which a concentration of the spider silk fibroin dissolved in the polar solvent is 15% to 70% by mass based on a mass of the protein solution.

According to another aspect of the present invention, there is provided a method for producing a molded article, including: obtaining a protein solution containing a protein and a polar solvent in which the protein is dissolved, by the above method; and removing the polar solvent from the protein solution to obtain a molded article containing the protein.

With this method, it is possible to easily produce a molded article of a protein using a high-concentration protein solution.

According to still another aspect of the present invention, there is provided a method for regenerating a protein from a molded article, including: applying a pressure to a polar solvent, which is in contact with the molded article containing the protein, to obtain a protein solution containing the protein and the polar solvent in which the protein is dissolved; and collecting the protein from the protein solution.

### Advantageous Effects of Invention

An aspect of the present invention can provide a method that makes it possible to easily obtain a high-concentration structural protein solution without requiring dialysis or the like.

### Brief Description of Drawings

FIG. 1 is a photograph showing an analysis result according to SDS-PAGE of a protein solution obtained in Example 4.
FIG. 2 is a photograph showing an analysis result according to SDS-PAGE of a protein solution obtained in Example 5.
FIG. 3 is a photograph showing an analysis result according to SDS-PAGE of protein solutions obtained in Examples 14 to 19.

### Description of Embodiments

Hereinafter, some embodiments of the present invention will be described in detail. However, the present invention is not limited to the following embodiments.

One embodiment of a method for producing a protein solution includes preparing a dispersion liquid containing a protein and a polar solvent in which the protein is dispersed, and applying a pressure to the dispersion liquid to obtain a protein solution containing the protein and the polar solvent in which the protein is dissolved.

By applying a pressure to a dispersion liquid containing a protein, it is possible to improve solubility of the protein in a solvent. As a result, it is possible to prepare a high-concentration protein solution even without performing a step such as dialysis. The present inventors have presumed that the above effect is obtained, because, by applying a pressure to a solution containing a protein, a solvent is caused to permeate into a crystalline portion of the protein which is poorly soluble and is composed of a β sheet, α helix, or the like, which is poor in solubility, or a portion of the protein which is aggregated at a high density by hydrogen bonding, these portions are loosened, and as a result, the protein is easily dissolved.

A dispersion liquid containing a protein can be prepared by mixing a protein in powder or any other form with a polar solvent. All of the protein contained in the dispersion liquid does not necessarily need to be dispersed in the polar solvent, and a part of the protein may be dissolved in the polar solvent.

Examples of the polar solvent used for preparing the dispersion liquid may include one or more solvents selected from the group consisting of water, alcohol, dimethylsulfoxide (DMSO), dimethylformamide (DMF), and hexafluoroacetone (HFA). From the viewpoint of obtaining a higher-concentration solution, the polar solvent can be dimethylsulfoxide alone, or a mixed solvent of dimethylsulfoxide and water. From the viewpoint of reducing adverse effects on environment, the polar solvent can be water. From the viewpoint of obtaining a protein solution under milder conditions, the polar solvent may contain alcohol. For example, the polar solvent may be a mixed solvent of water and alcohol, a mixed solvent of dimethylsulfoxide and alcohol, or a mixed solvent of water, alcohol, and dimethylsulfoxide. A proportion of the alcohol to a total amount of the polar solvent (or mixed solvent) may be 5% to 100% by mass, or 10% to 50% by mass. In a case where a polar solvent containing alcohol is used, there is a tendency that a protein can be dissolved in the polar solvent at a lower pressure.

In the present specification, the term "alcohol" means a compound composed of an aliphatic group that may have a substituent, and a hydroxyl group that is bonded to the aliphatic group. The aliphatic group may be, for example, substituted with a halogen atom such as a fluorine atom or may be unsubstituted. An example of a fluoroalcohol having an aliphatic group substituted with a fluorine atom is hexafluoroisopropanol (HFIP).

Alcohols having a low boiling point are particularly advantageous in that they allow conditions, under which preparation of an alcohol solution, concentration thereof, formation of a molded article, and the like are performed, to be mild. From such viewpoint, alcohols may have a boiling point of, for example, 99°C or lower and 50°C or higher under 1 atm. The alcohols may have a boiling point of 60°C or higher under 1 atm. Further, in general, alcohols having one hydroxyl group tend to have a lower boiling point than alcohols having two or more hydroxyl groups.

The number of carbons of the alcohol (number of carbons of the aliphatic group) is not particularly limited, and may be 1 to 10. From the viewpoint of obtaining a protein solution under particularly mild conditions, the number of carbons of the alcohol may be 2 to 8 or 2 to 5. The alcohol contained in the polar solvent may be at least one kind of alcohol having 1 to 10 carbon atoms selected from the group consisting of methanol, ethanol, propanol, butanol, pentanol, hexanol, heptanol, octanol, nonanol, decanol, and isomers of these alcohols; at least one kind of alcohol having 2 to 5 carbon atoms selected from the group consisting of ethanol, propanol, butanol, and isomers of these alcohols; or at least one kind of alcohol selected from the group consisting of ethanol, 1-propanol, and 2-propanol.

The protein may be a structural protein. The structural protein means a protein that forms or retains structure, morphology, and the like in vivo. Examples of the structural protein include hydrophobic proteins, and polypeptides prone to self-aggregation in polar solvents. Since these structural proteins are generally poorly soluble in polar solvents, the method of this embodiment is particularly useful for producing solutions of these structural proteins.

The structural protein may include one or more members selected from the group consisting of fibroin and keratin. The fibroin may be, for example, one or more members selected from the group consisting of silk fibroin, spider silk fibroin, and hornet silk fibroin. The structural protein may be silk fibroin, spider silk fibroin, or a combination thereof. In a case where the silk fibroin and the spider silk fibroin are used in combination, a proportion of the silk fibroin may be, for example, 40 parts by mass or less, 30 parts by mass or less, or 10 parts by mass or less, with respect to 100 parts by mass of the spider silk fibroin.

Silk is a fiber obtained from a cocoon of silkworm which is a larva of the silkworm moth (Bombyx mori). Generally, one cocoon filament is composed of two silk fibroins and a glue (sericin) covering them from the outside. The silk fibroin is composed of numerous fibrils. The silk fibroin is covered with four layers of sericin. Practically, silk filaments obtained by dissolving and removing an outer sericin by refinement are used for clothing applications. Common silk has a specific gravity of 1.33, a fineness of 3.3 decitex on average, and a fiber length of about 1,300 to 1,500 m. The silk fibroin is obtained from natural or domesticated silk cocoons, or from secondhand or discarded silk fabric, as raw materials.

The silk fibroin may be a sericin-removed silk fibroin, a sericin-unremoved silk fibroin, or a combination thereof. The sericin-removed silk fibroin is powders obtained by freeze-drying a silk fibroin which is purified by removing sericin covering the silk fibroin, other fats, and the like. The sericin-unremoved silk fibroin is an unpurified fibroin from which sericin and the like have not been removed.

The spider silk fibroin may contain a spider silk polypeptide selected from the group consisting of natural spider silk proteins and polypeptides derived from the natural spider silk proteins.

Examples of the natural spider silk protein include a major dragline silk protein, a weft yarn protein, and a minor ampullate gland protein. Since the major dragline silk has a repeated region composed of a crystalline region and an amorphous region, it is presumed to have both high stress and stretchability. A major feature of the weft yarn of the spider silk is that it has no crystalline region and has a repeated region composed of an amorphous region. On the other hand, the weft yarn is inferior in stress as compared with the major dragline silk, but has high stretchability. This is believed to be due to the fact that most of the weft yarns are composed of amorphous regions.

The major dragline silk protein is produced in the spider's major ampullate gland and has the feature of being excellent in toughness. Examples of the major dragline silk protein include the major ampullate gland spidroins MaSp1 and MaSp2 derived from Nephila clavipes, and ADF3 and ADF4 derived from Araneus diadematus. ADF3 is one of the two major silk ribbon thread proteins of Araneus diadematus. The polypeptide derived from the natural spider silk protein may be a polypeptide derived from these silk ribbon thread proteins. Polypeptides derived from ADF3 are relatively easy to synthesize and have excellent characteristics in terms of strength and toughness.

The weft yarn protein is produced in the spider's flagelliform gland. Examples of the weft yarn protein include flagelliform silk proteins derived from Nephila clavipes.

The polypeptide derived from the natural spider silk protein may be a recombinant spider silk protein. Examples of the recombinant spider silk protein include variants, analogs, or derivatives of the natural spider silk proteins. One suitable example of such polypeptide is a recombinant spider silk protein of the major dragline silk protein (also referred to as "a polypeptide derived from a major dragline silk protein").

The polypeptide derived from the major dragline silk protein may contain 2 or more, 5 or more, or 10 or more of a unit of an amino acid sequence (also referred to as a motif) represented by Formula 1: REP1-REP2 (1). An upper limit of the number of units of the amino acid sequence represented by Formula 1: REP1-REP2 (1), is not particularly limited, and may be, for example, 300 or less, or 200 or less. Alternatively, the polypeptide derived from the major dragline silk protein may be a polypeptide which contains the unit of the amino acid sequence represented by Formula 1: REP1-REP2 (1), and of which the C-terminal sequence is an amino acid sequence represented by any one of SEQ ID NOs: 1 to 3 or having homology of 90% or more with the amino acid sequence represented by any one of SEQ ID NOs: 1 to 3. In the polypeptides derived from the major dragline silk protein, the units of the amino acid sequence represented by Formula 1: REP1-REP2 (1), may be the same as or different from one another.

In Formula 1, a proportion of the number of alanine residues with respect to a total number of amino acid residues in the REP1 motif is typically 83% or more, and may be 86% or more, 90% or more, or 95% or more. REP1 may be a polyalanine of which a proportion of the number of alanine residues is 100%. Alanines (Ala) that are consecutively arranged in REP1 may be 2 residues or more, 3 residues or more, 4 residues or more, or 5 residues or more. In REP1, consecutively arranged alanines may be 20 residues or less, 16 residues or less, 12 residues or less, or 10 residues or less. The REP1 motif may contain other amino acid residues selected from serine (Ser), glycine (Gly), glutamine (Gln), and the like, in addition to alanine (Ala).

In Formula 1, REP2 is an amino acid sequence consisting of 10 to 200 amino acid residues, and a total residue number of glycine (Gly), serine (Ser), glutamine (Gln), and alanine (Ala) contained in the amino acid sequence may be 40% or more, 60% or more, or 70% or more with respect to a total number of amino acid residues.

In the major dragline silk, REP1 corresponds to a crystalline region forming a crystalline β sheet in the fiber, and REP 2 corresponds to an amorphous region, which is more flexible and most of which lacks an ordered structure, in the fiber. [REP1-REP2] corresponds to a repeated region (repetitive sequence) composed of a crystalline region and an amorphous region, and it is a characteristic sequence of the silk ribbon thread protein.

The amino acid sequence represented by SEQ ID NO: 1 is identical to an amino acid sequence consisting of 50 amino acid residues at the C-terminal of the amino acid sequence of ADF3 (NCBI Accession Number: AAC47010, GI: 1263287). The amino acid sequence represented by SEQ ID NO: 2 is identical to an amino acid sequence obtained by removing 20 residues from the C-terminal of the amino acid sequence represented by SEQ ID NO: 1. The amino acid sequence represented by SEQ ID NO: 3 is identical to an amino acid sequence obtained by removing 29 residues from the C-terminal of the amino acid sequence represented by SEQ ID NO: 1.

The polypeptide containing two or more units of the amino acid sequence represented by Formula 1: REP1-REP2 (1), can be, for example, a polypeptide consisting of the amino acid sequence represented by SEQ ID NO: 7. The polypeptide consisting of the amino acid sequence represented by SEQ ID NO: 7 is one obtained by mutating the amino acid sequence of ADF3 (NCBI Accession Number: AAC47010, GI: 1263287), to the N-terminal of which the amino acid sequence (SEQ ID NO: 4) consisting of a start codon, a His10 tag, and an HRV 3C protease (Human rhinovirus 3C protease) recognition site is added, so that translation thereof terminates at the 543rd amino acid residue.

The polypeptide containing two or more units of the amino acid sequence represented by Formula 1: REP1-REP2 (1), can be a protein which consists of an amino acid sequence obtained by substitution, deletion, insertion, and/or addition of one or a plurality of amino acids in the amino acid sequence represented by SEQ ID NO: 7 and has a repeated region composed of a crystalline region and an amorphous region. In the present specification, "one or a plurality of" means, for example, 1 to 40, 1 to 35, 1 to 30, 1 to 25, 1 to 20, 1 to 15, 1 to 10, and a range selected from one or several. In the present specification, "one or several" means 1 to 9, 1 to 8, 1 to 7, 1 to 6, 1 to 5, 1 to 4, 1 to 3, 1 to 2, or one.

The polypeptide containing two or more units of the amino acid sequence represented by Formula 1: REP1-REP2 (1), may be a recombinant protein derived from ADF 4 having the amino acid sequence represented by SEQ ID NO: 8. The amino acid sequence represented by SEQ ID NO: 8 is one obtained by adding the amino acid sequence (SEQ ID NO: 4) consisting of a start codon, a His10 tag, and an HRV 3C protease (Human rhinovirus 3C protease) recognition site to the N-terminal of a partial amino acid sequence of ADF 4 (NCBI Accession Number: AAC47011, GI: 1263289) obtained from the NCBI database.

The polypeptide containing two or more units of the amino acid sequence represented by Formula 1: REP1-REP2 (1), can be a polypeptide which consists of an amino acid sequence obtained by substitution, deletion, insertion, and/or addition of one or a plurality of amino acids in the amino acid sequence represented by SEQ ID NO: 8 and has a repeated region composed of a crystalline region and an amorphous region.

The polypeptide containing two or more units of the amino acid sequence represented by Formula 1: REP1-REP2 (1), may be a recombinant protein derived from MaSp2 having the amino acid sequence represented by SEQ ID NO: 9. The amino acid sequence represented by SEQ ID NO: 9 is one obtained by adding the amino acid sequence (SEQ ID NO: 4) consisting of a start codon, a His 10 tag, and an HRV 3C protease (Human rhinovirus 3C protease) recognition site to the N-terminal of a partial amino acid sequence of MaSp2 (NCBI Accession Number: AAT75313, GI: 50363147) obtained from the NCBI database.

The polypeptide containing two or more units of the amino acid sequence represented by Formula 1: REP1-REP2 (1), can be a polypeptide which consists of an amino acid sequence obtained by substitution, deletion, insertion, and/or addition of one or a plurality of amino acids in the amino acid sequence represented by SEQ ID NO: 9 and has a repeated region composed of a crystalline region and an amorphous region.

A polypeptide derived from the weft yarn protein may contain 10 or more, 20 or more, or 30 or more units of the amino acid sequence represented by Formula 2: REP3 (2). An upper limit of the number of units of the amino acid sequence represented by Formula 2: REP3 (2) is not particularly limited, and it may be, for example, 300 or less, or 200 or less.

In Formula 2, REP3 means an amino acid sequence consisting of Gly-Pro-Gly-Gly-X, in which X denotes one amino acid selected from the group consisting of alanine (Ala), serine (Ser), tyrosine (Tyr), and valine (Val).

A polypeptide containing 10 or more units of the amino acid sequence represented by Formula 2: REP3 (2), can be, for example, a recombinant protein derived from a flagelliform silk protein having the amino acid sequence represented by SEQ ID NO: 10. The amino acid sequence represented by SEQ ID NO: 10 is an amino acid sequence obtained by combining a repeat portion and an amino acid sequence (referred to as PR1 sequence) from the 1220th residue to the 1659th residue from the N-terminal which corresponds to a motif, of a partial sequence (NCBI Accession Number: AAF36090, GI: 7106224) of the flagelliform silk protein of Nephila clavipes obtained from the NCBI database, with a C-terminal amino acid sequence from the 816th residue to the 907th residue from the C-terminal of a partial sequence (NCBI Accession Number: AAC38847, GI: 2833649) of the flagelliform silk protein of Nephila clavipes obtained from the NCBI database, and adding, to the N-terminal of the combined sequence, the amino acid sequence (SEQ ID NO: 4) consisting of a start codon, a His10 tag, and an HRV 3C protease recognition site.

The polypeptide containing 10 or more units of the amino acid sequence represented by Formula 2: REP3 (2), can be a polypeptide which consists of an amino acid sequence obtained by substitution, deletion, insertion, and/or addition of one or a plurality of amino acids in the amino acid sequence represented by SEQ ID NO: 10 and has a repeated region composed of an amorphous region.

A molecular weight of the protein or polypeptide may be 500 kDa or less, 300 kDa or less, 200 kDa or less, or 100 kDa or less, and may be 10 kDa or more, from the viewpoint of productivity in a case where a recombinant protein is produced using a microorganism such as Escherichia coli as a host.

Hornet silk fibroin is a protein produced by bee larva and may contain a polypeptide selected from the group consisting of natural hornet silk proteins and polypeptides derived from the natural hornet silk proteins.

A polypeptide can be produced, for example, by using a host transformed with an expression vector containing a gene encoding the polypeptide.

A method for producing a gene encoding a polypeptide is not particularly limited. For example, in a case of a natural spider silk protein, a gene encoding the protein can be produced by a method of cloning the gene by amplification thereof from a spider-derived cell using polymerase chain reaction (PCR) or the like, or produced by chemical synthesis. The chemical synthesis method of the gene is also not particularly limited, and for example, the gene can be chemically synthesized by a method of linking oligonucleotides, which are automatically synthesized by AKTA oligopilot plus 10/100 (GE Healthcare Japan Co., Ltd) or the like based on amino acid sequence information of the natural spider silk protein obtained from the NCBI's web database, or the like, using PCR or the like. In this case, in order to facilitate purification and identification of the protein, a gene encoding a protein that consists of an amino acid sequence obtained by adding an amino acid sequence consisting of an start codon and a His10 tag to the N-terminal of the above amino acid sequence, may be synthesized.

As the expression vector, a plasmid, a phage, a virus, or the like capable of expressing a protein from a DNA sequence can be used. A plasmid-type expression vector is not particularly limited as long as it can cause a target gene to be expressed in a host cell and can be self-amplified. For example, in a case where Escherichia coli Rosetta (DE3) is used as a host, pET22b(+) plasmid vector, pCold plasmid vector, and the like can be used. Among these, pET22b(+) plasmid vector can be used from the viewpoint of protein productivity. As the host, for example, animal cells, plant cells, microorganisms, and the like can be used.

A combination of the above-mentioned structural protein such as silk fibroin and spider silk fibroin, and other proteins may be contained in a dispersion liquid and a protein solution obtained therefrom. Examples of other proteins include collagen, soy protein, casein, keratin, and whey protein. Physical properties derived from proteins can be adjusted by combining other proteins with the structural protein. A proportion of other proteins may be, for example, 40 parts by mass or less, 30 parts by mass or less, or 10 parts by mass or less, with respect to 100 parts by mass of the structural protein.

A concentration of the protein in the dispersion liquid can be 1% by mass or more, 15% by mass or more, 30% by mass or more, 40% by mass or more, or 50% by mass or more, based on a mass of the dispersion liquid. From the viewpoint of production efficiency of the protein solution, the concentration of the protein can be 70% by mass or less, 65% by mass or less, or 60% by mass or less, based on a mass of the dispersion liquid or protein solution.

The dispersion liquid may further contain one or two or more kinds of inorganic salts. By adding the inorganic salt to the dispersion liquid, an effect of solubility improvement by pressurization can be further remarkable. Examples of the inorganic salt include an inorganic salt formed of a Lewis acid and a Lewis base as shown below. The Lewis base may be, for example, an oxo acid ion (a nitrate ion, a perchlorate ion, or the like), a metal oxo acid ion (a permanganate ion or the like), a halide ion, a thiocyanate ion, a cyanate ion, or the like. The Lewis acid may be, for example, a metal ion such as an alkali metal ion or an alkaline earth metal ion, a polyatomic ion such as an ammonium ion, a complex ion, or the like. Specific examples of the inorganic salt include lithium salts such as lithium chloride, lithium bromide, lithium iodide, lithium nitrate, lithium perchlorate, and lithium thiocyanate; calcium salts such as calcium chloride, calcium bromide, calcium iodide, calcium nitrate, calcium perchlorate, and calcium thiocyanate; iron salts such as iron chloride, iron bromide, iron iodide, iron nitrate, iron perchlorate, and iron thiocyanate; aluminum salts such as aluminum chloride, aluminum bromide, aluminum iodide, aluminum nitrate, aluminum perchlorate, and aluminum thiocyanate; potassium salts such as potassium chloride, potassium bromide, potassium iodide, potassium nitrate, potassium perchlorate, and potassium thiocyanate; sodium salts such as sodium chloride, sodium bromide, sodium iodide, sodium nitrate, sodium nitrate, sodium perchlorate, and sodium thiocyanate; zinc salts such as zinc chloride, zinc bromide, zinc iodide, zinc nitrate, zinc perchlorate, and zinc thiocyanate; magnesium salts such as magnesium chloride, magnesium bromide, magnesium iodide, magnesium nitrate, magnesium perchlorate, and magnesium thiocyanate; barium salts such as barium chloride, barium bromide, barium iodide, barium nitrate, barium perchlorate, and barium thiocyanate; and strontium salts such as strontium chloride, strontium bromide, strontium iodide, strontium nitrate, strontium perchlorate, and strontium thiocyanate, and the like. A concentration of the inorganic salt can be 1.0% by mass or more, 5.0% by mass or more, 9.0% by mass or more, 15% by mass or more, or 20.0% by mass or more, based on a total amount of the protein. The concentration of the inorganic salt may also be 40% by mass or less, 35% by mass or less, or 30% by mass or less, based on the total amount of the protein.

The dispersion liquid and the protein solution may contain various additives as necessary. Examples of the additive include a plasticizer, a crystal nucleating agent, an antioxidant, an ultraviolet absorber, a colorant, a filler, and a synthetic resin. A concentration of the additive may be 50% by mass or less based on the total amount of the protein.

A predetermined pressure is applied to a dispersion liquid containing a protein and a polar solvent, so that the protein is dissolved in the polar solvent to produce a protein solution. By applying a pressure to the dispersion liquid, it is possible to dissolve the protein in the polar solvent even at a relatively low temperature. Therefore, it is possible to produce a high-concentration solution while suppressing induction of alteration, gelation, and degradation of the protein. Furthermore, since a concentration step using dialysis or the like is not necessarily required, it is possible to increase productivity of the protein solution.

The pressure applied to the dispersion liquid is adjusted so that the protein dissolves properly in the polar solvent, depending on types of the protein and the polar solvent, a desired concentration, and the like. In a case where the pressure applied to the dispersion liquid is high, a higher-concentration solution is easily obtained. From this viewpoint, the pressure applied to the dispersion liquid can be 0.05 MPa or more, 0.06 MPa or more, 0.07 MPa or more, 0.08 MPa or more, 0.1 MPa or more, 1.0 MPa or more, 5.0 MPa or more, or 10 MPa or more. The pressure applied to the dispersion liquid can be 300 MPa or less, 150 MPa or less, 50 MPa or less, or 30 MPa or less.

A method of applying a pressure to the dispersion liquid is not particularly limited. For example, it is possible to apply a pressure to the dispersion liquid by causing an inert gas such as nitrogen, argon, or the like, or air to be enclosed in a pressure-resistant container containing the dispersion liquid, and adjusting a pressure inside the pressure-resistant container with heating or the like.

Pressure may be applied to the dispersion liquid while the dispersion is being heated. The heating is not limited to the time during which the pressure is applied, and, for example, the pressure may be applied to the dispersion liquid after heating the dispersion liquid to a predetermined temperature. The heating temperature may be 150°C or lower, 140°C or lower, 135°C or lower, or 130°C or lower, from the viewpoint of further suppressing degradation of the protein. In a case where the polar solvent is water, from the viewpoint of further suppressing water-mediate degradation of the protein, the heating temperature is desirably 140°C or lower. From the viewpoint of obtaining a higher-concentration solution, the heating temperature may be 70°C or higher, 90°C or higher, or 100°C or higher. These upper and lower limits can be combined arbitrarily. For example, the heating temperature may be 70°C or higher and 150°C or lower, 90°C or higher and 140°C or lower, or 100°C or higher and 130°C or lower. The heating temperature may be a constant temperature or may vary.

A pressure may be applied to the dispersion liquid while the dispersion is being stirred. The stirring is not limited to the time during which the pressure is applied, and the dispersion liquid may be stirred before and after the application of the pressure. A stirring method is not particularly limited. For example, the dispersion liquid can be stirred with inclined blades, turbine blades, or the like.

A protein solution obtained after pressurization may contain gas for pressurization. Thus the method for producing a protein solution according to one embodiment may further include removing the gas from the protein solution. A method of removing the gas is not particularly limited, and examples thereof include a method using a centrifugal separator. By applying the protein solution to a centrifugal separator, it is possible to remove a layer containing a relatively large amount of gas.

Proteins obtained by the method of one embodiment can include proteins dissolved at a high concentration in a polar solvent. A concentration of the protein dissolved in the polar solvent of the protein solution can be the same as the concentration in the dispersion liquid. Specifically, based on a mass of the protein solution, the concentration of the protein dissolved in the polar solvent can be 1% by mass or more, 15% by mass or more, 30% by mass or more, 40% by mass or more, or 50% by mass or more, and can be 70% by mass or less, 65% by mass or less, or 60% by mass or less.

A protein solution containing a protein at a high concentration can be used, for example, to produce a molded article of the protein by various methods. A method for producing a molded article according to one embodiment may include removing a polar solvent from the protein solution to obtain a molded article containing the protein. According to this method, since it is possible to reduce the amount of solvent to be removed in the process of producing the molded article, production cost can be reduced. In particular, a protein solution containing spider silk fibroin at a high concentration can be used to produce a molded article having excellent physical properties in which characteristics of the spider silk fibroin are exhibited.

For example, by using a protein solution as a dope solution, it is possible to produce molded articles such as gels, films, fibers, and the like. The film can be produced, for example, by forming a film of a protein solution (dope solution) and removing a polar solvent from the formed film. The fiber can be produced, for example, by spinning a protein solution and removing a polar solvent from the spun protein solution.

By using the above-described method for producing a protein solution, it is also possible to regenerate proteins forming molded articles. One embodiment of a method for regenerating a protein from a molded article can include applying a pressure to a polar solvent, which is in contact with the molded article containing the protein, to obtain a protein solution containing the protein and the polar solvent in which the protein is dissolved, and collecting the protein from the protein solution.

The polar solvent used in the method for regenerating the protein and materials that can be added thereto can be selected in the same manner as the above-mentioned method for producing the protein solution. The molded article may be immersed in a polar solvent and a pressure may be applied to the polar solvent. Alternatively, a molded article which has been pulverized into particulates may be dispersed in a polar solvent to prepare a dispersion liquid, and a pressure may be applied to the dispersion liquid. Conditions such as pressure and heating can be set similarly to the above-described method for producing a protein solution.

A method for collecting a protein from a protein solution is not particularly limited, and a usual method such as a method of removing a polar solvent from a protein solution, reprecipitation, or the like can be applied. A protein in any form such as powder may be collected from a protein solution containing a protein eluted from a molded article, or a molded article may be directly produced by the above-described method.

### EXAMPLES

Hereinafter, the present invention will be described in more detail by way of examples. However, the present invention is not limited to these examples.

### Gene synthesis

### (1) Synthesis of Gene ADF3Kai

A partial amino acid sequence of ADF3 (NCBI Accession Number: AAC47010, GI: 1263287), that is one of two major silk ribbon thread proteins of Araneus diadematus, was obtained from the NCBI's web database, and synthesis of a gene encoding an amino acid sequence (SEQ ID NO: 5) that is obtained by adding, to the N-terminal of the partial amino acid sequence, an amino acid sequence (SEQ ID NO: 4) consisting of a start codon, a His10 tag, and an HRV 3C protease (Human rhinovirus 3C protease) recognition site, was entrusted to GenScript. As a result, a pUC57 vector, into which the gene ADF3Kai consisting of the nucleotide sequence represented by SEQ ID NO: 6 had been introduced, (the vector having a NdeI site immediately upstream of the 5' end of the gene and a XbaI site immediately downstream of the 5' end thereof) was obtained. Thereafter, the same gene was restriction enzyme-treated with NdeI and EcoRI, and recombined into a pET22b(+) expression vector.

### (2) Synthesis of gene ADF3Kai-noNR

By using the pET22b(+) vector, into which the gene ADF3Kai obtained above had been introduced, as a template, through site-directed mutagenesis using the PrimeStar Mutagenesis Basal Kit (manufactured by Takara Bio Inc.), codon GTG corresponding to valine (Val) which is a 543rd amino acid residue was mutated to a stop codon TAA in the amino acid sequence of ADF3Kai (SEQ ID NO: 5), and thus the gene ADF3Kai-noNR represented by SEQ ID NO: 11 was constructed on the pET22b(+). An accuracy of the mutagenesis was checked by a sequence reaction using the 3130xI Genetic Analyzer (Applied Biosystems). The amino acid sequence of ADF3Kai-noNR is represented by SEQ ID NO: 7.

### Expression of Protein

The pET22b (+) expression vector containing the gene sequence of ADF3Kai-noNR obtained above was transformed into Escherichia coli Rosetta (DE3). The obtained single colony was incubated for 15 hours in 2 mL of an LB culture medium containing ampicillin. Then, 1.4 mL of the same culture solution was added to 140 mL of an LB culture medium containing ampicillin, and incubated under a condition of 37°C and 200 rpm until an OD600 reached 3.5. Next, the culture solution with the OD600 of 3.5 was added to 7 L of a 2 x YT culture medium containing ampicillin, together with 140 mL of 50% glucose, and further incubated until an OD600 reached 4.0. Thereafter, isopropyl-p-thiogalactoviranoside (IPTG) was added to the obtained culture solution with the OD600 of 4.0 so that the final concentration thereof was 0.5 mM, thereby inducing expression of the protein. 2 hours after the IPTG addition, the culture solution was centrifuged and bacterial cells were collected. Protein solutions prepared from the culture solution before and after the IPTG addition were, respectively, electrophoresed in a polyacrylamide gel. Consequently, a band of a target size was observed depending on the addition of IPTG, and expression of a target protein was confirmed. Escherichia coli expressing the protein ADF3Kai-noNR was stored in a freezer (-20°C).

### Preparation Example of Spider Silk Polypeptide

(I) To a centrifuge tube (50 mL), about 4.5 g of bacterial cells of the Escherichia coli expressing the ADF3Kai-noNR protein and 30 mL of a buffer solution AI (20 mM Tris-HCl, pH 7.4) were added, and the bacterial cells were dispersed with a mixer ("SI-0286" manufactured by GE, level 10). Then, the dispersion was centrifuged (10,000 rpm, 10 minutes, room temperature) with a centrifugal separator ("MX-305" manufactured by Tommy Seiko Co., Ltd.), and the supernatant was discarded.
(II) To the precipitate (bacterial cells) obtained by the centrifugation, 30 mL of the buffer solution AI and 0.3 mL of 0.1 M PMSF (dissolved with isopropanol) were added, and the mixture was dispersed for 3 minutes with the above-mentioned mixer (level 10) manufactured by GE. Thereafter, the bacterial cells were disrupted using an ultrasonic disruptor ("VCX 500" manufactured by SONIC & MATERIALS INC) and centrifuged (10,000 rpm, 10 minutes, room temperature).
(III) To the precipitate obtained by the centrifugation, 30 mL of the buffer solution AI was added, and dispersed for 3 minutes with a mixer ("T18 Basic Ultra Turrax" manufactured by IKA, level 2). Then, the dispersion was centrifuged (10,000 rpm, 10 minutes, room temperature) with the above-mentioned centrifugal separator manufactured by Tommy Seiko Co., Ltd., and the supernatant was removed.
(IV) To the centrifuge tube from which the supernatant was discarded, a 7.5 M urea buffer solution I (7.5 M urea, 10 mM sodium dihydrogen phosphate, 20 mM NaCl, 1 mM Tris-HCl, pH 7.0) was added, and the precipitation was well dispersed with the above-mentioned ultrasonic disruptor manufactured by SMT (level 7). Thereafter, dissolution was performed with the above-mentioned shaker (200 rpm, 60°C) manufactured by TAITEC CORPORATION for 120 minutes. The protein solution after the dissolution was centrifuged (11,000 × g, 10 minutes, room temperature) with the above-mentioned centrifugal separator manufactured by Tommy Seiko Co., Ltd., and the supernatant was dialyzed against water using a dialysis tube (cellulose tube 36/32 of Sanko Junyaku Co., Ltd.). A white aggregated protein obtained after the dialysis was collected by centrifugation, and water was removed by a freeze dryer to collect freeze-dried powders. A purification degree of the target protein ADF3Kai-noNR in the obtained freeze-dried powders was checked by performing image analysis for the results of polyacrylamide gel electrophoresis (CBB staining) of the powders using Totallab (Nonlinear Dynamics Ltd.). As a result, the purification degree of ADF3Kai-noNR was about 85%.

### Examples 1 to 7, and Comparative Examples 1 to 3

### Dissolution Test of Spider Silk Polypeptide

Freeze-dried powders (molecular weight: 60 kDa) of the spider silk polypeptide (spider silk fibroin) obtained in the above "Preparation Example of Spider Silk Polypeptide", and water or dimethylsulfoxide (DMSO) were mixed at a ratio (parts by mass) as shown in Table 1 to prepare dispersion liquids in which the spider silk polypeptide was dispersed in water. As shown in Table 1, lithium chloride was added to a part of the dispersion liquids. The obtained dispersion liquids were placed in a pressure-resistant container, and the pressure-resistant container was sealed and then heated so that the temperature inside the container was 90°C. Furthermore, nitrogen was introduced into the pressure-resistant container so that the pressure inside the container was adjusted to a predetermined pressure of 1.0 to 10 MPa, and the pressure was applied to the dispersion liquids. After 2 hours, pressurization was stopped and the pressure-resistant container was cooled to 25°C. Thereafter, a dissolution state of the polypeptide was visually observed, and solubility was evaluated according to the following criteria. The results are shown in Table 1.
A: All polypeptides were dissolved.
B: A slight amount of undissolved polypeptides was observed.
C: Polypeptides were not dissolved.

### Analysis according to SDS-Polyacrylamide Gel Electrophoresis (SDS-PAGE)

The solution for which the spider silk polypeptide was confirmed to be dissolved was analyzed according to SDS-PAGE using sodium dodecyl sulfate (SDS). By performing image analysis for the results of electrophoresis under the following conditions, a degraded state of the dissolved polypeptide was checked. The results are shown in Table 1. In Table 1, "A" indicates that most of the structural proteins in the solution were not degraded, and "C" indicates that most of the structural proteins in the solution were degraded.

### SDS-PAGE Analysis Conditions

Power supply device: PowerPactm Universal (manufactured by BIO-RAD)
Image analysis system: Gel Doctm EZ Imager (manufactured by BIO-RAD)
Gel for electrophoresis: Mini-PROTEAN (R) TGX™ Gels (manufactured by BIO-RAD)
Molecular weight marker: XL-Ladder (manufactured by APRO SCOEMCE)
Fixative solution: Novex™ InVision™ His-Tag In-Gel Staining Kit (manufactured by Thermo Fisher Scientific)
Stain: Oriole™ Fluorescent Gel Stain (manufactured by BIO-RAD)

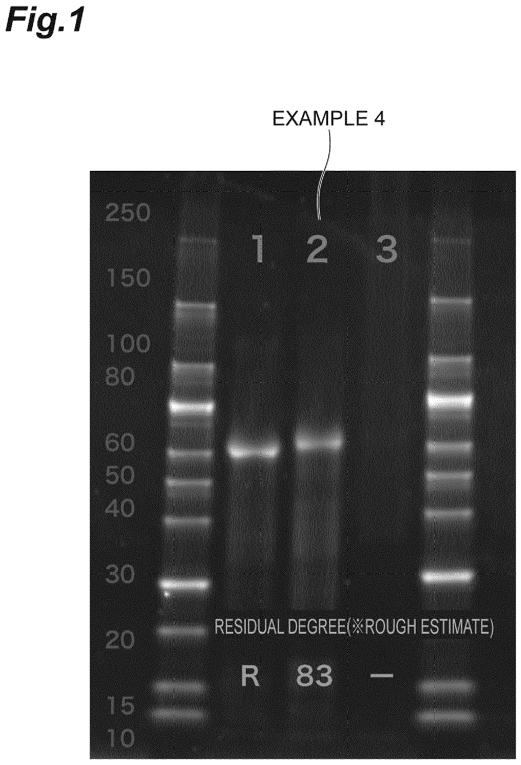

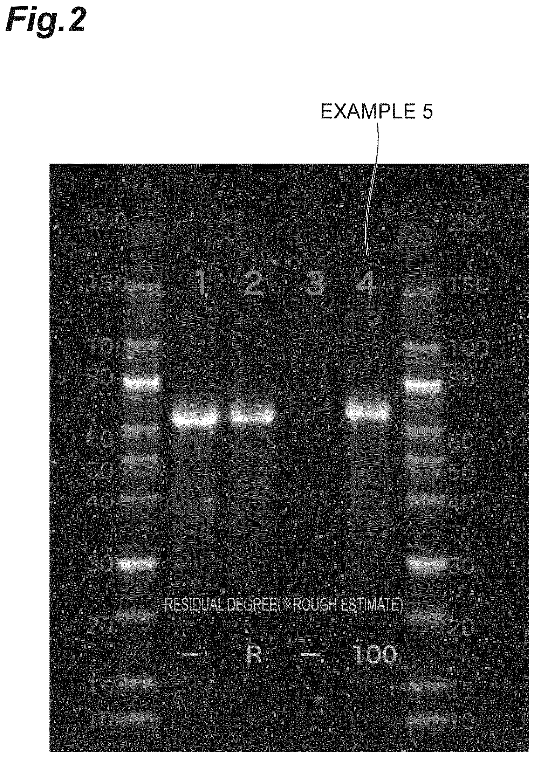

FIG. 1 and FIG. 2 are analysis results according to SDS-PAGE for the protein solutions obtained in Example 4 and Example 5, respectively. "R" in the drawings shows a reference test with a sample prepared by immersing freeze-dried powders of the spider silk polypeptide in water and then drying the same. A residual degree of the spider silk polypeptide estimated from comparison of the intensity of color development was about 83% in Example 4 and about 100% in Example 5.

### Examples 8 to 13, and Comparative Example 4

### Dissolution Test of Silk Fibroin

Silk fibroin derived from Bombyx mori (fibroin heavy chain Fib-H [Bombyx mori], Accession No. AAF 76983. Hereinafter, it is also simply referred to as "silk fibroin." A theoretical molecular weight: 391.5 kDa) and water were mixed at a ratio (parts by mass) as shown in Table 2 to prepare dispersion liquids in which the silk fibroin was dispersed in water. As shown in Table 2, lithium chloride was added to a part of the dispersion liquids. The obtained dispersion liquids were placed in a pressure-resistant container, and the pressure-resistant container was sealed and then heated so that the temperature inside the container was 90°C. Furthermore, nitrogen was introduced into the pressure-resistant container so that the pressure inside the container was adjusted to a predetermined pressure of 1.0 to 10 MPa, and the dispersion liquids were pressurized. After 48 hours, pressurization was stopped and the pressure-resistant container was cooled to 25°C. Thereafter, a dissolution state of the silk fibroin was visually observed, and solubility was evaluated based on the same criteria as for the spider silk fibroin. The results are shown in Table 2. Since the silk fibroin has a large molecular weight, it was difficult to perform analysis according to SDS-PAGE.

**Table 1**

| | | Example | | | | | | | Comp. Example | | |
|---|---|---|---|---|---|---|---|---|---|---|---|
| | | 1 | 2 | 3 | 4 | 5 | 6 | 7 | 1 | 2 | 3 |
| Protein | Spider silk fibroin | 1.0 | 1.0 | 1.0 | 30 | 40 | 13.0 | 13.0 | 1.0 | 13.0 | 13.0 |
| Polar solvent | Water | 99.0 | 99.0 | 99.0 | 70 | 40 | - | - | 99.0 | - | - |
| | DMSO | - | - | - | - | - | 87.0 | 87.0 | - | 87.0 | 87.0 |
| Inorganic salt | LiCl | - | - | - | - | 20 | - | - | - | - | - |
| Production condition | Pressure (/MPa) | 5.0 | 10.0 | 1.0 | 5.0 | 5.0 | 5.0 | 10.0 | - | - | - |
| | Temperature (/°C) | 90 | 90 | 90 | 110 | 110 | 70 | 70 | 90 | 90 | 150 |
| Evaluation item | Solubility evaluation | A | A | B | A | A | A | A | C | C | A |
| | SDS-PAGE | A | A | A | A | A | A | A | - | - | C |

**Table 2**

| | | Example | | | | | | Comp. Example |
|---|---|---|---|---|---|---|---|---|
| | | 8 | 9 | 10 | 11 | 12 | 13 | 4 |
| Protein | Silk fibroin | 0.1 | 0.1 | 0.1 | 0.1 | 0.1 | 0.1 | 0.1 |
| Polar solvent | Water | 99.9 | 99.9 | 99.9 | 79.9 | 79.9 | 79.9 | 99.9 |
| Inorganic salt | LiCl | - | - | - | 20.0 | 20.0 | 20.0 | - |
| Production condition | Pressure (/MPa) | 5.0 | 1.0 | 10.0 | 5.0 | 1.0 | 10.0 | - |
| | Temperature (/°C) | 90 | 90 | 90 | 90 | 90 | 90 | 90 |
| Evaluation item | Solubility evaluation | B | B | B | A | A | A | C |
| | SDS-PAGE | - | - | - | - | - | - | - |

As shown in Tables 1 and 2, it was confirmed that by applying a pressure to the dispersion liquid in which the structural protein is dispersed, it is possible to obtain a protein solution in which the structural protein is dissolved at a high concentration.

### Examples 14 to 19

### Dissolution Test 2 of Spider Silk Polypeptide

### (1) Synthesis of Nucleic Acid Encoding Modified Fibroin and Construction of Expression Vector

Based on the nucleotide sequence and amino acid sequence of Nephila clavipes (GenBank Accession Number: P46804.1, GI: 1174415), which is a naturally occurring spider silk fibroin, modified fibroins having the amino acid sequence represented by SEQ ID NOs: 12 and 13, respectively, were designed.

The amino acid sequence represented by SEQ ID NO: 12 was obtained as follows. Starting from the naturally occurring fibroin, in the amino acid sequence in which alanine residues in an (A)ₙ motif are consecutive to each other, deletion was performed so that the number of the consecutive alanine residues is 5; the (A)ₙ motif ((A)₅) was deleted at an interval of two motifs from the N-terminal side to the C-terminal side; one [(A)ₙ motif-REP] was inserted in front of the C-terminal sequence; and all GGX's in REP were substituted with GQX's. The amino acid sequence represented by SEQ ID NO: 13 was obtained by adding the amino acid sequence (tag sequence and hinge sequence) represented by SEQ ID NO: 14 to the N-terminal of the amino acid sequence represented by SEQ ID NO: 12.

A nucleic acid encoding a protein having the amino acid sequence represented by SEQ ID NO: 13 which was obtained by adding a His tag sequence and hinge sequence (SEQ ID NO: 14) to the N-terminal of the amino acid sequence represented by SEQ ID NO: 12, was synthesized. In the nucleic acid, an NdeI site was added to the 5' end thereof and an EcoRI site was added downstream of the stop codon thereof. The nucleic acid was cloned into a cloning vector (pUC 118). Thereafter, the same nucleic acid was excised by restriction enzyme-treatment with NdeI and EcoRI, and then recombined into a protein expression vector pET-22b(+) to obtain an expression vector.

### (2) Expression of Protein

Escherichia coli BLR (DE3) was transformed with a pET22b(+) expression vector containing a nucleic acid encoding a protein having the amino acid sequence represented by SEQ ID NO: 13. The transformed Escherichia coli was incubated for 15 hours in 2 mL of an LB culture medium containing ampicillin. By adding the same culture solution to 100mL of a culture medium for seed culture containing ampicillin (Table 3) so that OD₆₀₀ reached 0.005, the transformed Escherichia coli was inoculated. A temperature of the culture solution was kept at 30°C and flask incubation was performed (about 15 hours) until the OD₆₀₀ reached 5, and thus a seed culture solution was obtained.

**Table 3**

| Culture medium for seed culture (Per 1L at start of incubation | |
|---|---|
| Glucose | 5 g |
| KH₂PO₄ | 4 g |
| K₂HPO₄ | 10 g |
| Yeast Extract | 6 g |

The culture medium for seed culture was obtained by adding ampicillin so that a final concentration thereof was 100 mg/L

The seed culture medium was added to a jar fermentor to which 500 mL of a production culture medium (Table 4) had been added so that OD₆₀₀ reached 0.05, and the transformed E. coli was inoculated. A temperature of the culture solution was kept at 37°C, and incubation was performed under constant control at pH 6.9. A dissolved oxygen concentration in the culture solution was maintained at 20% of the dissolved oxygen saturation concentration.

**Table 4**

| Production culture medium (Per 1L at start of incubation) | |
|---|---|
| Glucose | 12 g |
| K₁HPO₄ | 9 g |
| MgSO₄·7H₂O | 2.4 g |
| Yeast Extract | 15 g |
| FeSO₄·7H₂O | 40 mg |
| MnSO₄·5H₂O | 40 mg |
| CaCl₂·2H₂O | 40 mg |
| GD-113 (Antifoaming agent) | 0.1 mL |

Immediately after the glucose in the production culture medium was completely consumed, a feed solution (glucose 455 g/1L, Yeast Extract 120g/1L) was added at a rate of 1 mL/min. A temperature of the culture solution was kept at 37°C, and incubation was performed under constant control at pH 6.9. A dissolved oxygen concentration in the culture solution was maintained at 20% of the dissolved oxygen saturation concentration, and incubation was performed for 20 hours. Thereafter, 1 M isopropyl-β-thiogalactopyranoside (IPTG) was added to the culture solution so that a final concentration thereof was 1 mM, to induce expression of a target protein. 20 hours after the IPTG addition, the culture solution was centrifuged to collect the bacterial cells. Using the bacterial cells prepared from the culture solution before and after the IPTG addition, SDS-PAGE was performed, and expression of the target protein was confirmed by appearance of a band of a target protein size depending on the IPTG addition.

### (3) Purification of Protein

The bacterial cells collected 2 hours after the IPTG addition were washed with 20 mM Tris-HCl buffer (pH 7.4). After the washing, the bacterial cells were suspended in 20 mM Tris-HCl buffer (pH 7.4) containing about 1 mM PMSF, and the cells were disrupted with a high-pressure homogenizer (GEA Niro Soavi). The disrupted cells were centrifuged to obtain a precipitate. The obtained precipitate was washed with 20 mM Tris-HCl buffer solution (pH 7.4) until a high purity was obtained. After the washing, the precipitate was suspended in 8 M guanidine buffer solution (8 M guanidine hydrochloride, 10 mM sodium dihydrogen phosphate, 20 mM NaCl, 1 mM Tris-HCl, pH 7.0) so that a concentration thereof was 100 mg/mL, and dissolved by stirring with a stirrer for 30 minutes at 60°C. After the dissolution, dialysis was performed against water using a dialysis tube (cellulose tube 36/32 of Sanko Junyaku Co., Ltd.). A white aggregated protein obtained after the dialysis was collected by centrifugation, and water was removed by a freeze dryer to collect freeze-dried powders.

A purification degree of the target protein in the obtained freeze-dried powders was checked by performing image analysis for the results of polyacrylamide gel electrophoresis of the powders using Totallab (Nonlinear Dynamics Ltd.). As a result, the purification degree of the protein was about 85%.

### (4) Dissolution test

Freeze-dried powders (molecular weight: 50 kDa) of the spider silk polypeptide (spider silk fibroin) obtained above, and water, ethanol, propanol, butanol, or Clynsolve P-7 (a mixed solvent containing 85.5 ± 1.0% of ethanol, less than 5.0% of isopropanol, 9.6 ± 0.5% of normal propanol, and 0.2% or less of water, "Clynsolve" is a registered trademark of Japan Alcohol Trading Company Limited) were mixed at a ratio (parts by mass) as shown in Table 5 to prepare dispersion liquids in which the spider silk polypeptide was dispersed in the liquid. The obtained dispersion liquid was placed in a pressure-resistant container, and the pressure-resistant container was sealed and then heated intermittently so that the temperature inside the container was maintained at a predetermined temperature of 90°C to 100°C. Increasing the temperature inside the container to 90 to 100°C makes the solvent in the dispersion liquid evaporate, and the pressure inside the container rises, thereby causing a pressure (calculated value) of 0.08 to 0.1 MPa to be applied to the dispersion liquid. The temperature inside the container was maintained at 90 to 100°C for 25 minutes from the start of heating, then a dissolution state of the polypeptide was visually observed, and solubility was evaluated according to the following criteria. The results are shown in Table 5.
A: All polypeptides were dissolved.
B: A slight amount of undissolved polypeptides was observed.
C: Polypeptides were not dissolved.

### (5) Analysis according to SDS-Polyacrylamide Gel Electrophoresis (SDS-PAGE)

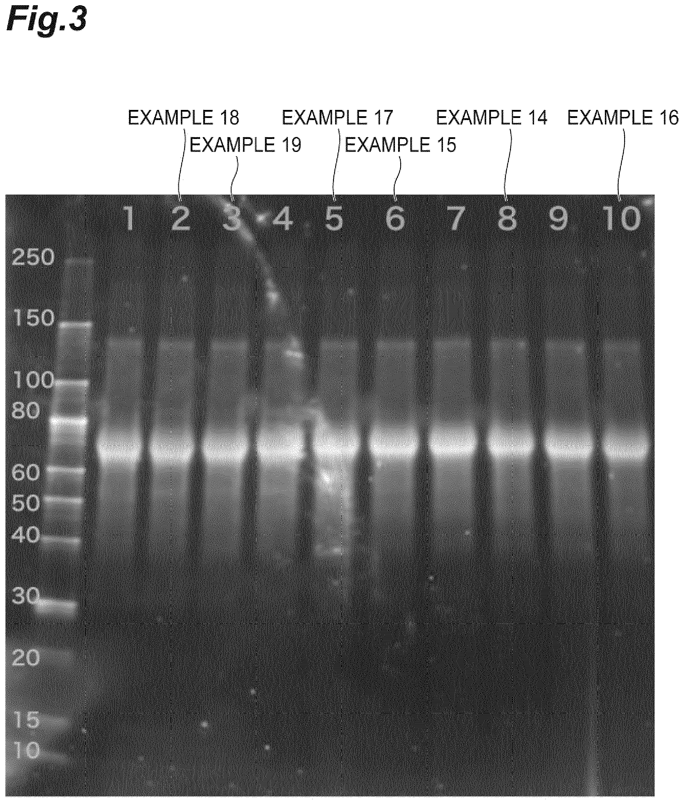

The solution for which the spider silk polypeptide was confirmed to be dissolved was analyzed according to SDS-PAGE under the same conditions as in Examples 1 to 7, and a degraded state of the dissolved polypeptide was checked. FIG. 3 shows the analysis results according to SDS-PAGE of the protein solutions obtained in Examples 14 to 19. The results are shown in Table 5. In Table 5, "A" indicates that most of the structural proteins in the solution were not degraded. In any of the Examples, a protein solution was obtained while suppressing protein degradation.

**Table 5**

| | | Example | | | | | |
|---|---|---|---|---|---|---|---|
| | | 14 | 15 | 16 | 17 | 18 | 19 |
| Protein | Spider silk fibroin | 25 | 23 | 25 | 25 | 23 | 25 |
| Polar solvent | Water | 52.5 | 53.9 | 52.5 | 52.5 | 53.9 | 60 |
| | Ethanol | 22.5 | 23.1 | - | - | - | - |
| | Propanol | - | - | 22.5 | - | - | - |
| | Butanol | - | - | - | 22.5 | - | - |
| | Mixed alcohol | - | - | - | - | 23.1 | 15 |
| Production condition | Pressure (/MPa) | 0.10 | 0.08 | 0.09 | 0.09 | 0.08 | 0.09 |
| | Temperature (/°C) | 95 | 90 | 95 | 100 | 90 | 95 |
| Evaluation item | Solubility evaluation | A | A | A | A | A | A |
| | SDS-PAGE | A | A | A | A | A | A |

As shown in Table 5, it was confirmed that by applying a pressure to the dispersion liquid in which the structural protein is dispersed, a protein solution, in which the structural protein is dissolved at a high concentration, is obtained.

## Claims

1. A method for producing a protein solution, comprising:
applying a pressure to a dispersion liquid containing a protein and a polar solvent in which the protein is dispersed, to obtain a protein solution containing the protein and the polar solvent in which the protein is dissolved.

2. The method according to claim 1,
wherein the pressure is 0.05 MPa or more.

3. The method according to claim 1 or 2,
wherein the protein is a structural protein.

4. The method according to claim 3,
wherein the structural protein is one or more structural proteins selected from the group consisting of fibroin and keratin.

5. The method according to claim 3,
wherein the structural protein is one or more fibroins selected from the group consisting of silk fibroin, spider silk fibroin, and hornet silk fibroin.

6. The method according to any one of claims 1 to 5,
wherein the pressure is applied to the dispersion liquid while the dispersion liquid is being heated.

7. The method according to any one of claims 1 to 6,
wherein the polar solvent includes one or more solvents selected from the group consisting of water, alcohol, dimethylsulfoxide, dimethylformamide, and hexafluoroacetone.

8. A protein solution comprising a spider silk fibroin and a polar solvent in which the spider silk fibroin is dissolved,
wherein a concentration of the spider silk fibroin dissolved in the polar solvent is 1% to 70% by mass based on a mass of the protein solution.

9. A method for producing a molded article, comprising:
obtaining a protein solution containing a protein and a polar solvent in which the protein is dissolved, by the method according to any one of claims 1 to 7; and
removing the polar solvent from the protein solution to obtain a molded article containing the protein.

10. A method for regenerating a protein from a molded article, comprising:
applying a pressure to a polar solvent, which is in contact with the molded article containing the protein, to obtain a protein solution containing the protein and the polar solvent in which the protein is dissolved; and
collecting the protein from the protein solution.

11. The method according to claim 10,
wherein the pressure is 0.05 MPa or more.

12. The method according to claim 10 or 11,
wherein the protein is a structural protein.

13. The method according to claim 12,
wherein the structural protein is one or more structural proteins selected from the group consisting of fibroin and keratin.

14. The method according to claim 12,
wherein the structural protein is one or more fibroins selected from the group consisting of silk fibroin, spider silk fibroin, and hornet silk fibroin.

15. The method according to any one of claims 10 to 14,
wherein the pressure is applied to the polar solvent while the polar solvent is being heated.

16. The method according to any one of claims 10 to 15,
wherein the polar solvent includes one or more solvents selected from the group consisting of water, alcohol, dimethylsulfoxide, dimethylformamide, and hexafluoroacetone.


### Sequence Listing

SEQUENCE LISTING

<110>  Spiber Inc.
       KOJIMA INDUSTRIES CORPORATION
       TEKUNOHAMA CO., LTD

<120>  METHOD OF PRODUCING PROTEIN SOLUTION

<130>  2015PS008WO1

<160>  14

<170>  PatentIn version 3.5

<210>  1
<211>  50
<212>  PRT
<213>  Araneus diadematus

<400>  1

Ser Gly Cys Asp Val Leu Val Gln Ala Leu Leu Glu Val Val Ser Ala
1               5                   10                  15

Leu Val Ser Ile Leu Gly Ser Ser Ser Ile Gly Gln Ile Asn Tyr Gly
            20                  25                  30

Ala Ser Ala Gln Tyr Thr Gln Met Val Gly Gln Ser Val Ala Gln Ala
        35                  40                  45

Leu Ala
    50

<210>  2
<211>  30
<212>  PRT
<213>  Araneus diadematus

<400>  2

Ser Gly Cys Asp Val Leu Val Gln Ala Leu Leu Glu Val Val Ser Ala
1               5                   10                  15

Leu Val Ser Ile Leu Gly Ser Ser Ser Ile Gly Gln Ile Asn
            20                  25                  30

<210>  3
<211>  21
<212>  PRT
<213>  Araneus diadematus

<400>  3

Ser Gly Cys Asp Val Leu Val Gln Ala Leu Leu Glu Val Val Ser Ala
1               5                   10                  15

Leu Val Ser Ile Leu
            20

<210>  4
<211>  24
<212>  PRT
<213>  Artificial Sequence

<220>
<223>  His tag and start codon

<400>  4

Met His His His His His His His His His His Ser Ser Gly Ser Ser
1               5                   10                  15

Leu Glu Val Leu Phe Gln Gly Pro
            20

<210>  5
<211>  660
<212>  PRT
<213>  Artificial Sequence

<220>
<223>  recombinant spider silk protein ADF3Kai

<400>  5

Met His His His His His His His His His His Ser Ser Gly Ser Ser
1               5                   10                  15

Leu Glu Val Leu Phe Gln Gly Pro Ala Arg Ala Gly Ser Gly Gln Gln
            20                  25                  30

Gly Pro Gly Gln Gln Gly Pro Gly Gln Gln Gly Pro Gly Gln Gln Gly
        35                  40                  45

Pro Tyr Gly Pro Gly Ala Ser Ala Ala Ala Ala Ala Ala Gly Gly Tyr
    50                  55                  60

Gly Pro Gly Ser Gly Gln Gln Gly Pro Ser Gln Gln Gly Pro Gly Gln
65                  70                  75                  80

Gln Gly Pro Gly Gly Gln Gly Pro Tyr Gly Pro Gly Ala Ser Ala Ala
                85                  90                  95

Ala Ala Ala Ala Gly Gly Tyr Gly Pro Gly Ser Gly Gln Gln Gly Pro
            100                 105                 110

Gly Gly Gln Gly Pro Tyr Gly Pro Gly Ser Ser Ala Ala Ala Ala Ala
        115                 120                 125

Ala Gly Gly Asn Gly Pro Gly Ser Gly Gln Gln Gly Ala Gly Gln Gln
    130                 135                 140

Gly Pro Gly Gln Gln Gly Pro Gly Ala Ser Ala Ala Ala Ala Ala Ala
145                 150                 155                 160

Gly Gly Tyr Gly Pro Gly Ser Gly Gln Gln Gly Pro Gly Gln Gln Gly
                165                 170                 175

Pro Gly Gly Gln Gly Pro Tyr Gly Pro Gly Ala Ser Ala Ala Ala Ala
            180                 185                 190

Ala Ala Gly Gly Tyr Gly Pro Gly Ser Gly Gln Gly Pro Gly Gln Gln
        195                 200                 205

Gly Pro Gly Gly Gln Gly Pro Tyr Gly Pro Gly Ala Ser Ala Ala Ala
    210                 215                 220

Ala Ala Ala Gly Gly Tyr Gly Pro Gly Ser Gly Gln Gln Gly Pro Gly
225                 230                 235                 240

Gln Gln Gly Pro Gly Gln Gln Gly Pro Gly Gly Gln Gly Pro Tyr Gly
                245                 250                 255

Pro Gly Ala Ser Ala Ala Ala Ala Ala Ala Gly Gly Tyr Gly Pro Gly
            260                 265                 270

Tyr Gly Gln Gln Gly Pro Gly Gln Gln Gly Pro Gly Gly Gln Gly Pro
        275                 280                 285

Tyr Gly Pro Gly Ala Ser Ala Ala Ser Ala Ala Ser Gly Gly Tyr Gly
    290                 295                 300

Pro Gly Ser Gly Gln Gln Gly Pro Gly Gln Gln Gly Pro Gly Gly Gln
305                 310                 315                 320

Gly Pro Tyr Gly Pro Gly Ala Ser Ala Ala Ala Ala Ala Ala Gly Gly
                325                 330                 335

Tyr Gly Pro Gly Ser Gly Gln Gln Gly Pro Gly Gln Gln Gly Pro Gly
            340                 345                 350

Gln Gln Gly Pro Gly Gln Gln Gly Pro Gly Gly Gln Gly Pro Tyr Gly
        355                 360                 365

Pro Gly Ala Ser Ala Ala Ala Ala Ala Ala Gly Gly Tyr Gly Pro Gly
    370                 375                 380

Ser Gly Gln Gln Gly Pro Gly Gln Gln Gly Pro Gly Gln Gln Gly Pro
385                 390                 395                 400

Gly Gln Gln Gly Pro Gly Gln Gln Gly Pro Gly Gln Gln Gly Pro Gly
                405                 410                 415

Gln Gln Gly Pro Gly Gln Gln Gly Pro Gly Gln Gln Gly Pro Gly Gly
            420                 425                 430

Gln Gly Ala Tyr Gly Pro Gly Ala Ser Ala Ala Ala Gly Ala Ala Gly
        435                 440                 445

Gly Tyr Gly Pro Gly Ser Gly Gln Gln Gly Pro Gly Gln Gln Gly Pro
    450                 455                 460

Gly Gln Gln Gly Pro Gly Gln Gln Gly Pro Gly Gln Gln Gly Pro Gly
465                 470                 475                 480

Gln Gln Gly Pro Gly Gln Gln Gly Pro Gly Gln Gln Gly Pro Tyr Gly
                485                 490                 495

Pro Gly Ala Ser Ala Ala Ala Ala Ala Ala Gly Gly Tyr Gly Pro Gly
            500                 505                 510

Ser Gly Gln Gln Gly Pro Gly Gln Gln Gly Pro Gly Gln Gln Gly Pro
        515                 520                 525

Gly Gly Gln Gly Pro Tyr Gly Pro Gly Ala Ala Ser Ala Ala Val Ser
    530                 535                 540

Val Gly Gly Tyr Gly Pro Gln Ser Ser Ser Val Pro Val Ala Ser Ala
545                 550                 555                 560

Val Ala Ser Arg Leu Ser Ser Pro Ala Ala Ser Ser Arg Val Ser Ser
                565                 570                 575

Ala Val Ser Ser Leu Val Ser Ser Gly Pro Thr Lys His Ala Ala Leu
            580                 585                 590

Ser Asn Thr Ile Ser Ser Val Val Ser Gln Val Ser Ala Ser Asn Pro
        595                 600                 605

Gly Leu Ser Gly Cys Asp Val Leu Val Gln Ala Leu Leu Glu Val Val
    610                 615                 620

Ser Ala Leu Val Ser Ile Leu Gly Ser Ser Ser Ile Gly Gln Ile Asn

625                 630                 635                 640

Tyr Gly Ala Ser Ala Gln Tyr Thr Gln Met Val Gly Gln Ser Val Ala
                645                 650                 655

Gln Ala Leu Ala
            660

<210>  6
<211>  1983
<212>  DNA
<213>  Artificial Sequence

<220>
<223>  recombinant DNA of spider silk protein gene ADF3Kai

<400>  6
atgcatcacc atcatcatca tcaccaccac cattcctcgg gctcatcctt ggaagtgtta      60

tttcaaggac cagcacgagc cggttcggga caacaagggc ctggccagca gggcccaggt     120

caacaagggc caggacagca gggtccttat gggcccggcg caagcgcagc agctgcggcc     180

gctggtggct atggtcctgg ctccggtcaa cagggccctt cgcaacaagg tcccgggcag     240

caaggtcctg gtggccaggg tccctacggg ccgggggcga gtgcggcagc agccgctgca     300

ggcggttatg gtccaggaag cggacagcaa ggtccgggag gtcaaggtcc gtatggccca     360

ggctctagcg cggctgccgc tgccgcgggt ggcaacggac cagggagcgg acaacagggc     420

gcgggacaac agggtccagg acagcaaggc ccaggggcgt cggcggctgc agcggcggcc     480

ggaggctatg gacccggctc aggacaacag ggaccgggtc aacaaggacc cggtggccaa     540

ggcccctatg gcccgggcgc cagcgcggcc gcagccgccg cgggcgggta cggccccggt     600

agcggccagg gaccaggtca gcaggggcca ggaggtcagg gcccatacgg tccgggcgca     660

tccgcggcgg cggcagcggc aggtggctac ggtcccggaa gcggccaaca ggggccaggg     720

caacaaggac caggacaaca aggtcctggg ggccaaggac cgtatggacc aggagcatca     780

gctgcagccg cggcagctgg cggttacggt ccaggctacg gccagcaggg tccgggtcag     840

cagggaccgg gaggccaggg gccttatggc cctggcgctt ccgcagccag tgccgcttct     900

ggaggatacg ggccgggaag cggtcagcaa ggccctggcc aacaaggacc tggaggccaa     960

gggccctacg gcccaggagc ctcggcagcc gcagctgccg caggtgggta tgggccaggt    1020

agcgggcaac aagggccggg tcagcaagga ccggggcaac agggacctgg gcagcaagga    1080

cccgggggtc aaggcccgta cggacctggt gcgtctgcag ctgctgctgc ggctggtgga    1140

tatggtccgg gatcggggca gcagggtccc ggtcagcagg gccctggtca gcaagggcca    1200

ggccaacagg gacccggaca acaaggcccg ggtcaacagg gtcctggaca gcaggggccg    1260

ggccaacaag gccctgggca acagggtccg gggggacagg gggcctatgg gcctggcgca    1320

tctgccgccg ctggcgcagc cggtgggtac gggcctgggt caggtcaaca ggggcctggt 1380

caacaaggcc ccgggcaaca gggccccggc cagcaaggtc cagggcagca gggcccggga 1440

cagcaagggc ctggacaaca ggggcccgga cagcagggac cttacgggcc cggtgcgagc 1500

gcagcggccg ccgccgcagg gggatatggc cccggatcgg gccagcaggg accaggccag 1560

caaggacctg gccaacaggg cccggggggt caggggccgt atggtcccgg cgctgcaagt 1620

gctgcagtgt ccgttggagg ttacggccct cagtcttcgt ctgttccggt ggcgtccgca 1680

gttgcgagta gactgtcttc acctgctgct tcatcgcgag tatcgagcgc tgtttcgtct 1740

cttgtctcgt cgggtcccac gaaacatgcc gccctttcaa atacgatttc atctgtagtg 1800

tcccaagtta gtgcaagtaa cccggggtta tccggatgcg acgttctcgt tcaggcactc 1860

ctagaagtag tatccgcgtt ggtgagcatc ttaggcagct cctcgatagg tcaaataaac 1920

tatggtgctt cagcccagta tacacagatg gtgggacaga gcgtcgcgca ggcattggct 1980

taa 1983

<210> 7
<211> 542
<212> PRT
<213> Artificial Sequence

<220>
<223> recombinant DNA of spider silk protein gene ADF3Kai

<400> 7

Met His His His His His His His His His His Ser Ser Gly Ser Ser
1               5                   10                  15

Leu Glu Val Leu Phe Gln Gly Pro Ala Arg Ala Gly Ser Gly Gln Gln
            20                  25                  30

Gly Pro Gly Gln Gln Gly Pro Gly Gln Gln Gly Pro Gly Gln Gln Gly
        35                  40                  45

Pro Tyr Gly Pro Gly Ala Ser Ala Ala Ala Ala Ala Ala Gly Gly Tyr
    50                  55                  60

Gly Pro Gly Ser Gly Gln Gln Gly Pro Ser Gln Gln Gly Pro Gly Gln
65                  70                  75                  80

Gln Gly Pro Gly Gly Gln Gly Pro Tyr Gly Pro Gly Ala Ser Ala Ala
                85                  90                  95

Ala Ala Ala Ala Gly Gly Tyr Gly Pro Gly Ser Gly Gln Gln Gly Pro
            100                 105                 110

Gly Gly Gln Gly Pro Tyr Gly Pro Gly Ser Ser Ala Ala Ala Ala Ala
        115                 120                 125

Ala Gly Gly Asn Gly Pro Gly Ser Gly Gln Gln Gly Ala Gly Gln Gln
    130                 135                 140

Gly Pro Gly Gln Gln Gly Pro Gly Ala Ser Ala Ala Ala Ala Ala Ala
145                 150                 155                 160

Gly Gly Tyr Gly Pro Gly Ser Gly Gln Gln Gly Pro Gly Gln Gln Gly
                165                 170                 175

Pro Gly Gly Gln Gly Pro Tyr Gly Pro Gly Ala Ser Ala Ala Ala Ala
            180                 185                 190

Ala Ala Gly Gly Tyr Gly Pro Gly Ser Gly Gln Gly Pro Gly Gln Gln
        195                 200                 205

Gly Pro Gly Gly Gln Gly Pro Tyr Gly Pro Gly Ala Ser Ala Ala Ala
    210                 215                 220

Ala Ala Ala Gly Gly Tyr Gly Pro Gly Ser Gly Gln Gln Gly Pro Gly
225                 230                 235                 240

Gln Gln Gly Pro Gly Gln Gln Gly Pro Gly Gly Gln Gly Pro Tyr Gly
                245                 250                 255

Pro Gly Ala Ser Ala Ala Ala Ala Ala Ala Gly Gly Tyr Gly Pro Gly
            260                 265                 270

Tyr Gly Gln Gln Gly Pro Gly Gln Gln Gly Pro Gly Gly Gln Gly Pro
        275                 280                 285

Tyr Gly Pro Gly Ala Ser Ala Ala Ser Ala Ala Ser Gly Gly Tyr Gly
    290                 295                 300

Pro Gly Ser Gly Gln Gln Gly Pro Gly Gln Gln Gly Pro Gly Gly Gln
305                 310                 315                 320

Gly Pro Tyr Gly Pro Gly Ala Ser Ala Ala Ala Ala Ala Ala Gly Gly
                325                 330                 335

Tyr Gly Pro Gly Ser Gly Gln Gln Gly Pro Gly Gln Gln Gly Pro Gly
            340                 345                 350

Gln Gln Gly Pro Gly Gln Gln Gly Pro Gly Gly Gln Gly Pro Tyr Gly
        355                 360                 365

Pro Gly Ala Ser Ala Ala Ala Ala Ala Ala Gly Gly Tyr Gly Pro Gly
    370                 375                 380

Ser Gly Gln Gln Gly Pro Gly Gln Gln Gly Pro Gly Gln Gln Gly Pro
385                 390                 395                 400

Gly Gln Gln Gly Pro Gly Gln Gln Gly Pro Gly Gln Gln Gly Pro Gly
                405                 410                 415

Gln Gln Gly Pro Gly Gln Gln Gly Pro Gly Gln Gln Gly Pro Gly Gly
            420                 425                 430

Gln Gly Ala Tyr Gly Pro Gly Ala Ser Ala Ala Ala Gly Ala Ala Gly
        435                 440                 445

Gly Tyr Gly Pro Gly Ser Gly Gln Gln Gly Pro Gly Gln Gln Gly Pro
    450                 455                 460

Gly Gln Gln Gly Pro Gly Gln Gln Gly Pro Gly Gln Gln Gly Pro Gly
465                 470                 475                 480

Gln Gln Gly Pro Gly Gln Gln Gly Pro Gly Gln Gln Gly Pro Tyr Gly
                485                 490                 495

Pro Gly Ala Ser Ala Ala Ala Ala Ala Ala Gly Gly Tyr Gly Pro Gly
            500                 505                 510

Ser Gly Gln Gln Gly Pro Gly Gln Gln Gly Pro Gly Gln Gln Gly Pro
        515                 520                 525

Gly Gly Gln Gly Pro Tyr Gly Pro Gly Ala Ala Ser Ala Ala
    530                 535                 540

<210>  8
<211>  434
<212>  PRT
<213>  Artificial

<220>
<223>  Recombinang spider silk protein ADF4Kai

<400>  8
   Met His His His His His His His His His His Ser Ser Gly Ser Ser
   1               5                   10                  15
   Leu Glu Val Leu Phe Gln Gly Pro Ala Gly Ser Ser Ala Ala Ala Ala
               20                  25                  30
   Ala Ala Ala Ser Gly Ser Gly Gly Tyr Gly Pro Glu Asn Gln Gly Pro
           35                  40                  45
   Ser Gly Pro Val Ala Tyr Gly Pro Gly Gly Pro Val Ser Ser Ala Ala
       50                  55                  60
   Ala Ala Ala Ala Ala Gly Ser Gly Pro Gly Gly Tyr Gly Pro Glu Asn
   65                  70                  75                  80
   Gln Gly Pro Ser Gly Pro Gly Gly Tyr Gly Pro Gly Gly Ser Gly Ser
                   85                  90                  95
   Ser Ala Ala Ala Ala Ala Ala Ala Ala Ser Gly Pro Gly Gly Tyr Gly
               100                 105                 110
   Pro Gly Ser Gln Gly Pro Ser Gly Pro Gly Gly Ser Gly Gly Tyr Gly
           115                 120                 125
   Pro Gly Ser Gln Gly Ala Ser Gly Pro Gly Gly Pro Gly Ala Ser Ala
       130                 135                 140
   Ala Ala Ala Ala Ala Ala Ala Ala Ala Ser Gly Pro Gly Gly Tyr Gly
   145                 150                 155                 160
   Pro Gly Ser Gln Gly Pro Ser Gly Pro Gly Ala Tyr Gly Pro Gly Gly
                   165                 170                 175
   Pro Gly Ser Ser Ala Ala Ala Ala Ala Ala Ala Ala Ser Gly Pro Gly
               180                 185                 190
   Gly Tyr Gly Pro Gly Ser Gln Gly Pro Ser Gly Pro Gly Val Tyr Gly
           195                 200                 205
   Pro Gly Gly Pro Gly Ser Ser Ala Ala Ala Ala Ala Ala Ala Gly Ser
       210                 215                 220
   Gly Pro Gly Gly Tyr Gly Pro Glu Asn Gln Gly Pro Ser Gly Pro Gly
   225                 230                 235                 240
   Gly Tyr Gly Pro Gly Gly Ser Gly Ser Ser Ala Ala Ala Ala Ala Ala
                   245                 250                 255
   Ala Ala Ser Gly Pro Gly Gly Tyr Gly Pro Gly Ser Gln Gly Pro Ser
               260                 265                 270
   Gly Pro Gly Gly Ser Gly Gly Tyr Gly Pro Gly Ser Gln Gly Gly Ser
           275                 280                 285
   Gly Pro Gly Ala Ser Ala Ala Ala Ala Ala Ala Ala Ala Ser Gly Pro
       290                 295                 300
   Gly Gly Tyr Gly Pro Gly Ser Gln Gly Pro Ser Gly Pro Gly Tyr Gln
   305                 310                 315                 320
   Gly Pro Ser Gly Pro Gly Ala Tyr Gly Pro Ser Pro Ser Ala Ser Ala
                   325                 330                 335
   Ser Val Ala Ala Ser Val Tyr Leu Arg Leu Gln Pro Arg Leu Glu Val
               340                 345                 350
   Ser Ser Ala Val Ser Ser Leu Val Ser Ser Gly Pro Thr Asn Gly Ala
           355                 360                 365
   Ala Val Ser Gly Ala Leu Asn Ser Leu Val Ser Gln Ile Ser Ala Ser
       370                 375                 380
   Asn Pro Gly Leu Ser Gly Cys Asp Ala Leu Val Gln Ala Leu Leu Glu
   385                 390                 395                 400
   Leu Val Ser Ala Leu Val Ala Ile Leu Ser Ser Ala Ser Ile Gly Gln
                   405                 410                 415
   Val Asn Val Ser Ser Val Ser Gln Ser Thr Gln Met Ile Ser Gln Ala
               420                 425                 430
   Leu Ser

<210>  9
<211>  355
<212>  PRT
<213>  Artificial

<220>
<223>  Recombinant spider silk protein MaSp2_N

<400>  9

Met His His His His His His Ser Ser Gly Ser Ser Leu Glu Val Leu
1               5                   10                  15

Phe Gln Gly Pro Ala Arg Ala Gly Pro Gly Gly Tyr Arg Pro Gly Gln
            20                  25                  30

Gln Gly Pro Ser Gly Pro Gly Ser Ala Ala Ala Ala Ala Ala Ala Ala
        35                  40                  45

Ala Gly Pro Gly Gly Tyr Gly Pro Gly Gln Gln Gly Pro Gly Gly Tyr
    50                  55                  60

Gly Pro Gly Gln Gln Gly Pro Ser Gly Ala Gly Ser Ala Ala Ala Ala
65                  70                  75                  80

Ala Ala Ala Gly Pro Gly Gln Gln Gly Leu Gly Gly Tyr Gly Pro Gly
                85                  90                  95

Gln Gln Gly Pro Gly Gly Tyr Gly Pro Gly Gln Gln Gly Pro Gly Gly
            100                 105                 110

Tyr Gly Pro Gly Ser Ala Ser Ala Ala Ala Ala Ala Ala Gly Pro Gly
        115                 120                 125

Gln Gln Gly Pro Gly Gly Tyr Gly Pro Gly Gln Gln Gly Pro Ser Gly
    130                 135                 140

Pro Gly Ser Ala Ser Ala Ala Ala Ala Ala Ala Gly Pro Gly Gly Tyr
145                 150                 155                 160

Gly Pro Gly Gln Gln Gly Pro Gly Gly Tyr Ala Pro Gly Gln Gln Gly
                165                 170                 175

Pro Ser Gly Pro Gly Ser Ala Ala Ala Ala Ala Ala Ala Ala Arg Ala
            180                 185                 190

Gly Pro Gly Gly Tyr Gly Pro Ala Gln Gln Gly Pro Ser Gly Pro Gly
        195                 200                 205

Ile Ala Ala Ser Ala Ala Ser Ala Gly Pro Gly Gly Tyr Gly Pro Ala
    210                 215                 220

Gln Gln Gly Pro Ala Gly Tyr Gly Pro Gly Ser Ala Val Ala Ala Ser
225                 230                 235                 240

Ala Gly Ala Gly Ser Ala Gly Tyr Gly Pro Gly Ser Gln Ala Ser Ala
                245                 250                 255

Ala Ala Ser Arg Leu Ala Ser Pro Asp Ser Gly Ala Arg Val Ala Ser
            260                 265                 270

Ala Val Ser Asn Leu Val Ser Ser Gly Pro Thr Ser Ser Ala Ala Leu
        275                 280                 285

Ser Ser Val Ile Ser Asn Ala Val Ser Gln Ile Gly Ala Ser Asn Pro
    290                 295                 300

Gly Leu Ser Gly Cys Asp Val Leu Ile Gln Ala Leu Leu Glu Ile Val
305                 310                 315                 320

Ser Ala Cys Val Thr Ile Leu Ser Ser Ser Ser Ile Gly Gln Val Asn
                325                 330                 335

Tyr Gly Ala Ala Ser Gln Phe Ala Gln Val Val Gly Gln Ser Val Leu
            340                 345                 350

Ser Ala Phe
        355

<210>  10
<211>  559
<212>  PRT
<213>  Artificial

<220>
<223>  Recombinant spider silk protein Flag_92_short2

<400>  10

Met His His His His His His His His His His Ser Ser Gly Ser Ser
1               5                   10                  15

Leu Glu Val Leu Phe Gln Gly Pro Gly Ala Gly Gly Ser Gly Pro Gly
            20                  25                  30

Gly Ala Gly Pro Gly Gly Val Gly Pro Gly Gly Ser Gly Pro Gly Gly
        35                  40                  45

Val Gly Pro Gly Gly Ser Gly Pro Gly Gly Val Gly Pro Gly Gly Ser
    50                  55                  60

Gly Pro Gly Gly Val Gly Pro Gly Gly Ala Gly Gly Pro Tyr Gly Pro
65                  70                  75                  80

Gly Gly Ser Gly Pro Gly Gly Ala Gly Gly Ala Gly Gly Pro Gly Gly
                85                  90                  95

Ala Tyr Gly Pro Gly Gly Ser Tyr Gly Pro Gly Gly Ser Gly Gly Pro
            100                 105                 110

Gly Gly Ala Gly Gly Pro Tyr Gly Pro Gly Gly Glu Gly Pro Gly Gly
        115                 120                 125

Ala Gly Gly Pro Tyr Gly Pro Gly Gly Ala Gly Gly Pro Tyr Gly Pro
    130                 135                 140

Gly Gly Ala Gly Gly Pro Tyr Gly Pro Gly Gly Glu Gly Gly Pro Tyr
145                 150                 155                 160

Gly Pro Gly Gly Ser Tyr Gly Pro Gly Gly Ala Gly Gly Pro Tyr Gly
                165                 170                 175

Pro Gly Gly Pro Tyr Gly Pro Gly Gly Glu Gly Pro Gly Gly Ala Gly
            180                 185                 190

Gly Pro Tyr Gly Pro Gly Gly Val Gly Pro Gly Gly Gly Gly Pro Gly
        195                 200                 205

Gly Tyr Gly Pro Gly Gly Ala Gly Pro Gly Gly Tyr Gly Pro Gly Gly
    210                 215                 220

Ser Gly Pro Gly Gly Tyr Gly Pro Gly Gly Ser Gly Pro Gly Gly Tyr
225                 230                 235                 240

Gly Pro Gly Gly Ser Gly Pro Gly Gly Tyr Gly Pro Gly Gly Ser Gly
                245                 250                 255

Pro Gly Gly Tyr Gly Pro Gly Gly Ser Gly Pro Gly Gly Ser Gly Pro
            260                 265                 270

Gly Gly Tyr Gly Pro Gly Gly Ser Gly Pro Gly Gly Ser Gly Pro Gly
        275                 280                 285

Gly Tyr Gly Pro Gly Gly Ser Gly Pro Gly Gly Tyr Gly Pro Gly Gly
    290                 295                 300

Ser Gly Pro Gly Gly Ser Gly Pro Gly Gly Tyr Gly Pro Gly Gly Ser
305                 310                 315                 320

Gly Pro Gly Gly Ser Gly Pro Gly Gly Tyr Gly Pro Gly Gly Ser Gly
                325                 330                 335

Pro Gly Gly Phe Gly Pro Gly Gly Phe Gly Pro Gly Gly Ser Gly Pro
            340                 345                 350

Gly Gly Tyr Gly Pro Gly Gly Ser Gly Pro Gly Gly Ala Gly Pro Gly
        355                 360                 365

Gly Val Gly Pro Gly Gly Phe Gly Pro Gly Gly Ala Gly Pro Gly Gly
    370                 375                 380

Ala Gly Pro Gly Gly Ala Gly Pro Gly Gly Ala Gly Pro Gly Gly Ala
385                 390                 395                 400

Gly Pro Gly Gly Ala Gly Pro Gly Gly Ala Gly Pro Gly Gly Ala Gly
                405                 410                 415

Pro Gly Gly Ala Gly Gly Ala Gly Gly Ala Gly Gly Ala Gly Gly Ser
            420                 425                 430

Gly Gly Ala Gly Gly Ser Gly Gly Thr Thr Ile Ile Glu Asp Leu Asp
        435                 440                 445

Ile Thr Ile Asp Gly Ala Asp Gly Pro Ile Thr Ile Ser Glu Glu Leu
    450                 455                 460

Thr Ile Ser Ala Tyr Tyr Pro Ser Ser Arg Val Pro Asp Met Val Asn
465                 470                 475                 480

Gly Ile Met Ser Ala Met Gln Gly Ser Gly Phe Asn Tyr Gln Met Phe
                485                 490                 495

Gly Asn Met Leu Ser Gln Tyr Ser Ser Gly Ser Gly Thr Cys Asn Pro
            500                 505                 510

Asn Asn Val Asn Val Leu Met Asp Ala Leu Leu Ala Ala Leu His Cys
        515                 520                 525

Leu Ser Asn His Gly Ser Ser Ser Phe Ala Pro Ser Pro Thr Pro Ala
    530                 535                 540

Ala Met Ser Ala Tyr Ser Asn Ser Val Gly Arg Met Phe Ala Tyr
545                 550                 555

<210>  11
<211>  1629
<212>  DNA
<213>  Artificial Sequence

<220>
<223>  recombinant spider silk protein gene ADF3Kai-noNR

<400>  11
atgcatcacc atcatcatca tcaccaccac cattcctcgg gctcatcctt ggaagtgtta      60

tttcaaggac cagcacgagc cggttcggga caacaagggc ctggccagca gggcccaggt     120

caacaagggc caggacagca gggtccttat gggcccggcg caagcgcagc agctgcggcc     180

gctggtggct atggtcctgg ctccggtcaa cagggccctt cgcaacaagg tcccgggcag     240

caaggtcctg gtggccaggg tccctacggg ccgggggcga gtgcggcagc agccgctgca     300

ggcggttatg gtccaggaag cggacagcaa ggtccgggag gtcaaggtcc gtatggccca     360

ggctctagcg cggctgccgc tgccgcgggt ggcaacggac cagggagcgg acaacagggc 420

gcgggacaac agggtccagg acagcaaggc ccaggggcgt cggcggctgc agcggcggcc 480

ggaggctatg gacccggctc aggacaacag ggaccgggtc aacaaggacc cggtggccaa 540

ggcccctatg gcccgggcgc cagcgcggcc gcagccgccg cgggcgggta cggccccggt 600

agcggccagg gaccaggtca gcaggggcca ggaggtcagg gcccatacgg tccgggcgca 660

tccgcggcgg cggcagcggc aggtggctac ggtcccggaa gcggccaaca ggggccaggg 720

caacaaggac caggacaaca aggtcctggg ggccaaggac cgtatggacc aggagcatca 780

gctgcagccg cggcagctgg cggttacggt ccaggctacg gccagcaggg tccgggtcag 840

cagggaccgg gaggccaggg gccttatggc cctggcgctt ccgcagccag tgccgcttct 900

ggaggatacg ggccgggaag cggtcagcaa ggccctggcc aacaaggacc tggaggccaa 960

gggccctacg gcccaggagc ctcggcagcc gcagctgccg caggtgggta tgggccaggt 1020

agcgggcaac aagggccggg tcagcaagga ccggggcaac agggacctgg gcagcaagga 1080

cccgggggtc aaggcccgta cggacctggt gcgtctgcag ctgctgctgc ggctggtgga 1140

tatggtccgg gatcggggca gcagggtccc ggtcagcagg gccctggtca gcaagggcca 1200

ggccaacagg gacccggaca acaaggcccg ggtcaacagg gtcctggaca gcaggggccg 1260

ggccaacaag gccctgggca acagggtccg gggggacagg gggcctatgg gcctggcgca 1320

tctgccgccg ctggcgcagc cggtgggtac gggcctgggt caggtcaaca ggggcctggt 1380

caacaaggcc ccgggcaaca gggccccggc cagcaaggtc cagggcagca gggcccggga 1440

cagcaagggc ctggacaaca ggggcccgga cagcagggac cttacgggcc cggtgcgagc 1500

gcagcggccg ccgccgcagg gggatatggc cccggatcgg gccagcaggg accaggccag 1560

caaggacctg gccaacaggg cccggggggt caggggccgt atggtcccgg cgctgcaagt 1620

gctgcataa 1629

<210> 12
<211> 590
<212> PRT
<213> Artificial Sequence

<220>
<223> Met_CRY1_L_A5_giza_QQQ (GizaQ)

<400> 12

Met Gly Pro Gly Gln Gln Gly Pro Tyr Gly Pro Gly Ala Ser Ala Ala
1               5                   10                  15

Ala Ala Ala Gly Gln Asn Gly Pro Gly Ser Gly Gln Gln Gly Pro Gly
            20                  25                  30

Gln Ser Gly Gln Tyr Gly Pro Gly Gln Gln Gly Pro Gly Gln Gln Gly
        35                  40                  45

Pro Gly Ser Ser Ala Ala Ala Ala Ala Gly Pro Gly Gln Tyr Gly Pro
    50                  55                  60

Gly Gln Gln Gly Pro Ser Ala Ser Ala Ala Ala Ala Ala Gly Pro Gly
65                  70                  75                  80

Ser Gly Gln Gln Gly Pro Gly Ala Ser Gly Gln Tyr Gly Pro Gly Gln
                85                  90                  95

Gln Gly Pro Gly Gln Gln Gly Pro Gly Ser Ser Ala Ala Ala Ala Ala
            100                 105                 110

Gly Gln Tyr Gly Ser Gly Pro Gly Gln Gln Gly Pro Tyr Gly Ser Ala
        115                 120                 125

Ala Ala Ala Ala Gly Pro Gly Ser Gly Gln Tyr Gly Gln Gly Pro Tyr
    130                 135                 140

Gly Pro Gly Ala Ser Gly Pro Gly Gln Tyr Gly Pro Gly Gln Gln Gly
145                 150                 155                 160

Pro Ser Ala Ser Ala Ala Ala Ala Ala Gly Ser Gly Gln Gln Gly Pro
                165                 170                 175

Gly Gln Tyr Gly Pro Tyr Ala Ser Ala Ala Ala Ala Ala Gly Gln Tyr
            180                 185                 190

Gly Ser Gly Pro Gly Gln Gln Gly Pro Tyr Gly Pro Gly Gln Ser Gly
        195                 200                 205

Ser Gly Gln Gln Gly Pro Gly Gln Gln Gly Pro Tyr Ala Ser Ala Ala
    210                 215                 220

Ala Ala Ala Gly Pro Gly Gln Gln Gly Pro Tyr Gly Pro Gly Ser Ser
225                 230                 235                 240

Ala Ala Ala Ala Ala Gly Gln Tyr Gly Tyr Gly Pro Gly Gln Gln Gly
                245                 250                 255

Pro Tyr Gly Pro Gly Ala Ser Gly Gln Asn Gly Pro Gly Ser Gly Gln
            260                 265                 270

Tyr Gly Pro Gly Gln Gln Gly Pro Gly Gln Ser Ala Ala Ala Ala Ala
        275                 280                 285

Gly Pro Gly Gln Gln Gly Pro Tyr Gly Pro Gly Ala Ser Ala Ala Ala
    290                 295                 300

Ala Ala Gly Gln Tyr Gly Pro Gly Gln Gln Gly Pro Gly Gln Tyr Gly
305                 310                 315                 320

Pro Gly Ser Ser Gly Pro Gly Gln Gln Gly Pro Tyr Gly Pro Gly Ser
                325                 330                 335

Ser Ala Ala Ala Ala Ala Gly Gln Tyr Gly Pro Gly Gln Gln Gly Pro
            340                 345                 350

Tyr Gly Pro Gly Gln Ser Ala Ala Ala Ala Ala Gly Gln Tyr Gln Gln
        355                 360                 365

Gly Pro Gly Gln Gln Gly Pro Tyr Gly Pro Gly Ala Ser Gly Pro Gly
    370                 375                 380

Gln Gln Gly Pro Tyr Gly Pro Gly Ala Ser Ala Ala Ala Ala Ala Gly
385                 390                 395                 400

Pro Gly Gln Tyr Gly Pro Gly Gln Gln Gly Pro Ser Ala Ser Ala Ala
                405                 410                 415

Ala Ala Ala Gly Gln Tyr Gly Ser Gly Pro Gly Gln Tyr Gly Pro Tyr
            420                 425                 430

Gly Pro Gly Gln Ser Gly Pro Gly Ser Gly Gln Gln Gly Gln Gly Pro
        435                 440                 445

Tyr Gly Pro Gly Ala Ser Ala Ala Ala Ala Ala Gly Gln Tyr Gly Pro
    450                 455                 460

Gly Gln Gln Gly Pro Tyr Gly Pro Gly Gln Ser Ala Ala Ala Ala Ala
465                 470                 475                 480

Gly Pro Gly Ser Gly Gln Tyr Gly Pro Gly Ala Ser Gly Gln Asn Gly
                485                 490                 495

Pro Gly Ser Gly Gln Tyr Gly Pro Gly Gln Gln Gly Pro Gly Gln Ser
            500                 505                 510

Ala Ala Ala Ala Ala Gly Gln Tyr Gln Gln Gly Pro Gly Gln Gln Gly
        515                 520                 525

Pro Tyr Gly Pro Gly Ala Ser Ala Ala Ala Ala Ala Gly Gln Tyr Gly
    530                 535                 540

Ser Gly Pro Gly Gln Gln Gly Pro Tyr Gly Pro Gly Gln Ser Gly Ser
545                 550                 555                 560

Gly Gln Gln Gly Pro Gly Gln Gln Gly Pro Tyr Ala Ser Ala Ala Ala
                565                 570                 575

Ala Ala Gly Pro Gly Ser Gly Gln Gln Gly Pro Gly Ala Ser
            580                 585                 590

<210>  13
<211>  601
<212>  PRT
<213>  Artificial Sequence

<220>
<223>  CRY1_L_A5_giza_QQQ (GizaQ)

<400>  13

Met His His His His His His Ser Ser Gly Ser Ser Gly Pro Gly Gln
1               5                   10                  15

Gln Gly Pro Tyr Gly Pro Gly Ala Ser Ala Ala Ala Ala Ala Gly Gln
            20                  25                  30

Asn Gly Pro Gly Ser Gly Gln Gln Gly Pro Gly Gln Ser Gly Gln Tyr
        35                  40                  45

Gly Pro Gly Gln Gln Gly Pro Gly Gln Gln Gly Pro Gly Ser Ser Ala
    50                  55                  60

Ala Ala Ala Ala Gly Pro Gly Gln Tyr Gly Pro Gly Gln Gln Gly Pro
65                  70                  75                  80

Ser Ala Ser Ala Ala Ala Ala Ala Gly Pro Gly Ser Gly Gln Gln Gly
                85                  90                  95

Pro Gly Ala Ser Gly Gln Tyr Gly Pro Gly Gln Gln Gly Pro Gly Gln
            100                 105                 110

Gln Gly Pro Gly Ser Ser Ala Ala Ala Ala Ala Gly Gln Tyr Gly Ser
        115                 120                 125

Gly Pro Gly Gln Gln Gly Pro Tyr Gly Ser Ala Ala Ala Ala Ala Gly
    130                 135                 140

Pro Gly Ser Gly Gln Tyr Gly Gln Gly Pro Tyr Gly Pro Gly Ala Ser
145                 150                 155                 160

Gly Pro Gly Gln Tyr Gly Pro Gly Gln Gln Gly Pro Ser Ala Ser Ala
                165                 170                 175

Ala Ala Ala Ala Gly Ser Gly Gln Gln Gly Pro Gly Gln Tyr Gly Pro
            180                 185                 190

Tyr Ala Ser Ala Ala Ala Ala Ala Gly Gln Tyr Gly Ser Gly Pro Gly
        195                 200                 205

Gln Gln Gly Pro Tyr Gly Pro Gly Gln Ser Gly Ser Gly Gln Gln Gly
    210                 215                 220

Pro Gly Gln Gln Gly Pro Tyr Ala Ser Ala Ala Ala Ala Ala Gly Pro
225                 230                 235                 240

Gly Gln Gln Gly Pro Tyr Gly Pro Gly Ser Ser Ala Ala Ala Ala Ala
                245                 250                 255

Gly Gln Tyr Gly Tyr Gly Pro Gly Gln Gln Gly Pro Tyr Gly Pro Gly
            260                 265                 270

Ala Ser Gly Gln Asn Gly Pro Gly Ser Gly Gln Tyr Gly Pro Gly Gln
        275                 280                 285

Gln Gly Pro Gly Gln Ser Ala Ala Ala Ala Ala Gly Pro Gly Gln Gln
    290                 295                 300

Gly Pro Tyr Gly Pro Gly Ala Ser Ala Ala Ala Ala Ala Gly Gln Tyr
305                 310                 315                 320

Gly Pro Gly Gln Gln Gly Pro Gly Gln Tyr Gly Pro Gly Ser Ser Gly
                325                 330                 335

Pro Gly Gln Gln Gly Pro Tyr Gly Pro Gly Ser Ser Ala Ala Ala Ala
            340                 345                 350

Ala Gly Gln Tyr Gly Pro Gly Gln Gln Gly Pro Tyr Gly Pro Gly Gln
        355                 360                 365

Ser Ala Ala Ala Ala Ala Gly Gln Tyr Gln Gln Gly Pro Gly Gln Gln
    370                 375                 380

Gly Pro Tyr Gly Pro Gly Ala Ser Gly Pro Gly Gln Gln Gly Pro Tyr
385                 390                 395                 400

Gly Pro Gly Ala Ser Ala Ala Ala Ala Ala Gly Pro Gly Gln Tyr Gly
                405                 410                 415

Pro Gly Gln Gln Gly Pro Ser Ala Ser Ala Ala Ala Ala Ala Gly Gln
            420                 425                 430

Tyr Gly Ser Gly Pro Gly Gln Tyr Gly Pro Tyr Gly Pro Gly Gln Ser
        435                 440                 445

Gly Pro Gly Ser Gly Gln Gln Gly Gln Gly Pro Tyr Gly Pro Gly Ala
    450                 455                 460

Ser Ala Ala Ala Ala Ala Gly Gln Tyr Gly Pro Gly Gln Gln Gly Pro
465                 470                 475                 480

Tyr Gly Pro Gly Gln Ser Ala Ala Ala Ala Ala Gly Pro Gly Ser Gly
                485                 490                 495

Gln Tyr Gly Pro Gly Ala Ser Gly Gln Asn Gly Pro Gly Ser Gly Gln
            500                 505                 510

Tyr Gly Pro Gly Gln Gln Gly Pro Gly Gln Ser Ala Ala Ala Ala Ala
        515                 520                 525

Gly Gln Tyr Gln Gln Gly Pro Gly Gln Gln Gly Pro Tyr Gly Pro Gly
    530                 535                 540

Ala Ser Ala Ala Ala Ala Ala Gly Gln Tyr Gly Ser Gly Pro Gly Gln
545                 550                 555                 560

Gln Gly Pro Tyr Gly Pro Gly Gln Ser Gly Ser Gly Gln Gln Gly Pro
                565                 570                 575

Gly Gln Gln Gly Pro Tyr Ala Ser Ala Ala Ala Ala Ala Gly Pro Gly
            580                 585                 590

Ser Gly Gln Gln Gly Pro Gly Ala Ser
        595                 600

<210>  14
<211>  12
<212>  PRT
<213>  Artificial Sequence

<220>
<223>  HisTag

<400>  14

Met His His His His His His Ser Ser Gly Ser Ser
1               5                   10

SEQUENCE LISTING

<110>  Spiber Inc.
       KOJIMA INDUSTRIES CORPORATION
       TEKUNOHAMA CO., LTD

<120>  METHOD OF PRODUCING PROTEIN SOLUTION

<130>  FP16-0846-00

<160>  14

<170>  PatentIn version 3.5

<210>  1
<211>  50
<212>  PRT
<213>  Araneus diadematus

<400>  1

Ser Gly Cys Asp Val Leu Val Gln Ala Leu Leu Glu Val Val Ser Ala
1               5                   10                  15

Leu Val Ser Ile Leu Gly Ser Ser Ser Ile Gly Gln Ile Asn Tyr Gly
            20                  25                  30

Ala Ser Ala Gln Tyr Thr Gln Met Val Gly Gln Ser Val Ala Gln Ala
        35                  40                  45

Leu Ala
    50

<210>  2
<211>  30
<212>  PRT
<213>  Araneus diadematus

<400>  2

Ser Gly Cys Asp Val Leu Val Gln Ala Leu Leu Glu Val Val Ser Ala
1               5                   10                  15

Leu Val Ser Ile Leu Gly Ser Ser Ser Ile Gly Gln Ile Asn
            20                  25                  30

<210>  3
<211>  21
<212>  PRT
<213>  Araneus diadematus

<400>  3

Ser Gly Cys Asp Val Leu Val Gln Ala Leu Leu Glu Val Val Ser Ala
1               5                   10                  15

Leu Val Ser Ile Leu
            20

<210>  4
<211>  24
<212>  PRT
<213>  Artificial Sequence

<220>
<223>  His tag and start codon

<400>  4

Met His His His His His His His His His His Ser Ser Gly Ser Ser
1               5                   10                  15

Leu Glu Val Leu Phe Gln Gly Pro
            20

<210>  5
<211>  660
<212>  PRT
<213>  Artificial Sequence

<220>
<223>  recombinant spider silk protein ADF3Kai

<400>  5

Met His His His His His His His His His His Ser Ser Gly Ser Ser
1               5                   10                  15

Leu Glu Val Leu Phe Gln Gly Pro Ala Arg Ala Gly Ser Gly Gln Gln
            20                  25                  30

Gly Pro Gly Gln Gln Gly Pro Gly Gln Gln Gly Pro Gly Gln Gln Gly
        35                  40                  45

Pro Tyr Gly Pro Gly Ala Ser Ala Ala Ala Ala Ala Ala Gly Gly Tyr
    50                  55                  60

Gly Pro Gly Ser Gly Gln Gln Gly Pro Ser Gln Gln Gly Pro Gly Gln
65                  70                  75                  80

Gln Gly Pro Gly Gly Gln Gly Pro Tyr Gly Pro Gly Ala Ser Ala Ala
                85                  90                  95

Ala Ala Ala Ala Gly Gly Tyr Gly Pro Gly Ser Gly Gln Gln Gly Pro
            100                 105                 110

Gly Gly Gln Gly Pro Tyr Gly Pro Gly Ser Ser Ala Ala Ala Ala Ala
        115                 120                 125

Ala Gly Gly Asn Gly Pro Gly Ser Gly Gln Gln Gly Ala Gly Gln Gln
    130                 135                 140

Gly Pro Gly Gln Gln Gly Pro Gly Ala Ser Ala Ala Ala Ala Ala Ala
145                 150                 155                 160

Gly Gly Tyr Gly Pro Gly Ser Gly Gln Gln Gly Pro Gly Gln Gln Gly
                165                 170                 175

Pro Gly Gly Gln Gly Pro Tyr Gly Pro Gly Ala Ser Ala Ala Ala Ala
            180                 185                 190

Ala Ala Gly Gly Tyr Gly Pro Gly Ser Gly Gln Gly Pro Gly Gln Gln
        195                 200                 205

Gly Pro Gly Gly Gln Gly Pro Tyr Gly Pro Gly Ala Ser Ala Ala Ala
    210                 215                 220

Ala Ala Ala Gly Gly Tyr Gly Pro Gly Ser Gly Gln Gln Gly Pro Gly
225                 230                 235                 240

Gln Gln Gly Pro Gly Gln Gln Gly Pro Gly Gly Gln Gly Pro Tyr Gly
                245                 250                 255

Pro Gly Ala Ser Ala Ala Ala Ala Ala Ala Gly Gly Tyr Gly Pro Gly
            260                 265                 270

Tyr Gly Gln Gln Gly Pro Gly Gln Gln Gly Pro Gly Gly Gln Gly Pro
        275                 280                 285

Tyr Gly Pro Gly Ala Ser Ala Ala Ser Ala Ala Ser Gly Gly Tyr Gly
    290                 295                 300

Pro Gly Ser Gly Gln Gln Gly Pro Gly Gln Gln Gly Pro Gly Gly Gln
305                 310                 315                 320

Gly Pro Tyr Gly Pro Gly Ala Ser Ala Ala Ala Ala Ala Ala Gly Gly
                325                 330                 335

Tyr Gly Pro Gly Ser Gly Gln Gln Gly Pro Gly Gln Gln Gly Pro Gly
            340                 345                 350

Gln Gln Gly Pro Gly Gln Gln Gly Pro Gly Gly Gln Gly Pro Tyr Gly
        355                 360                 365

Pro Gly Ala Ser Ala Ala Ala Ala Ala Ala Gly Gly Tyr Gly Pro Gly
    370                 375                 380

Ser Gly Gln Gln Gly Pro Gly Gln Gln Gly Pro Gly Gln Gln Gly Pro
385                 390                 395                 400

Gly Gln Gln Gly Pro Gly Gln Gln Gly Pro Gly Gln Gln Gly Pro Gly
                405                 410                 415

Gln Gln Gly Pro Gly Gln Gln Gly Pro Gly Gln Gln Gly Pro Gly Gly
            420                 425                 430

Gln Gly Ala Tyr Gly Pro Gly Ala Ser Ala Ala Ala Gly Ala Ala Gly
        435                 440                 445

Gly Tyr Gly Pro Gly Ser Gly Gln Gln Gly Pro Gly Gln Gln Gly Pro
    450                 455                 460

Gly Gln Gln Gly Pro Gly Gln Gln Gly Pro Gly Gln Gln Gly Pro Gly
465                 470                 475                 480

Gln Gln Gly Pro Gly Gln Gln Gly Pro Gly Gln Gln Gly Pro Tyr Gly
                485                 490                 495

Pro Gly Ala Ser Ala Ala Ala Ala Ala Ala Gly Gly Tyr Gly Pro Gly
            500                 505                 510

Ser Gly Gln Gln Gly Pro Gly Gln Gln Gly Pro Gly Gln Gln Gly Pro
        515                 520                 525

Gly Gly Gln Gly Pro Tyr Gly Pro Gly Ala Ala Ser Ala Ala Val Ser
    530                 535                 540

Val Gly Gly Tyr Gly Pro Gln Ser Ser Ser Val Pro Val Ala Ser Ala
545                 550                 555                 560

Val Ala Ser Arg Leu Ser Ser Pro Ala Ala Ser Ser Arg Val Ser Ser
                565                 570                 575

Ala Val Ser Ser Leu Val Ser Ser Gly Pro Thr Lys His Ala Ala Leu
            580                 585                 590

Ser Asn Thr Ile Ser Ser Val Val Ser Gln Val Ser Ala Ser Asn Pro
        595                 600                 605

Gly Leu Ser Gly Cys Asp Val Leu Val Gln Ala Leu Leu Glu Val Val
    610                 615                 620

Ser Ala Leu Val Ser Ile Leu Gly Ser Ser Ser Ile Gly Gln Ile Asn

625                 630                 635                 640

Tyr Gly Ala Ser Ala Gln Tyr Thr Gln Met Val Gly Gln Ser Val Ala
                645                 650                 655

Gln Ala Leu Ala
            660

<210>  6
<211>  1983
<212>  DNA
<213>  Artificial Sequence

<220>
<223>  recombinant DNA of spider silk protein gene ADF3Kai

<400>  6
atgcatcacc atcatcatca tcaccaccac cattcctcgg gctcatcctt ggaagtgtta      60

tttcaaggac cagcacgagc cggttcggga caacaagggc ctggccagca gggcccaggt     120

caacaagggc caggacagca gggtccttat gggcccggcg caagcgcagc agctgcggcc     180

gctggtggct atggtcctgg ctccggtcaa cagggccctt cgcaacaagg tcccgggcag     240

caaggtcctg gtggccaggg tccctacggg ccgggggcga gtgcggcagc agccgctgca     300

ggcggttatg gtccaggaag cggacagcaa ggtccgggag gtcaaggtcc gtatggccca     360

ggctctagcg cggctgccgc tgccgcgggt ggcaacggac cagggagcgg acaacagggc     420

gcgggacaac agggtccagg acagcaaggc ccaggggcgt cggcggctgc agcggcggcc     480

ggaggctatg gacccggctc aggacaacag ggaccgggtc aacaaggacc cggtggccaa     540

ggcccctatg gcccgggcgc cagcgcggcc gcagccgccg cgggcgggta cggccccggt     600

agcggccagg gaccaggtca gcaggggcca ggaggtcagg gcccatacgg tccgggcgca     660

tccgcggcgg cggcagcggc aggtggctac ggtcccggaa gcggccaaca ggggccaggg     720

caacaaggac caggacaaca aggtcctggg ggccaaggac cgtatggacc aggagcatca     780

gctgcagccg cggcagctgg cggttacggt ccaggctacg gccagcaggg tccgggtcag     840

cagggaccgg gaggccaggg gccttatggc cctggcgctt ccgcagccag tgccgcttct     900

ggaggatacg ggccgggaag cggtcagcaa ggccctggcc aacaaggacc tggaggccaa     960

gggccctacg gcccaggagc ctcggcagcc gcagctgccg caggtgggta tgggccaggt    1020

agcgggcaac aagggccggg tcagcaagga ccggggcaac agggacctgg gcagcaagga    1080

cccgggggtc aaggcccgta cggacctggt gcgtctgcag ctgctgctgc ggctggtgga    1140

tatggtccgg gatcggggca gcagggtccc ggtcagcagg gccctggtca gcaagggcca    1200

ggccaacagg gacccggaca acaaggcccg ggtcaacagg gtcctggaca gcaggggccg    1260

ggccaacaag gccctgggca acagggtccg gggggacagg gggcctatgg gcctggcgca    1320

tctgccgccg ctggcgcagc cggtgggtac gggcctgggt caggtcaaca ggggcctggt 1380

caacaaggcc ccgggcaaca gggccccggc cagcaaggtc cagggcagca gggcccggga 1440

cagcaagggc ctggacaaca ggggcccgga cagcagggac cttacgggcc cggtgcgagc 1500

gcagcggccg ccgccgcagg gggatatggc cccggatcgg gccagcaggg accaggccag 1560

caaggacctg gccaacaggg cccggggggt caggggccgt atggtcccgg cgctgcaagt 1620

gctgcagtgt ccgttggagg ttacggccct cagtcttcgt ctgttccggt ggcgtccgca 1680

gttgcgagta gactgtcttc acctgctgct tcatcgcgag tatcgagcgc tgtttcgtct 1740

cttgtctcgt cgggtcccac gaaacatgcc gccctttcaa atacgatttc atctgtagtg 1800

tcccaagtta gtgcaagtaa cccggggtta tccggatgcg acgttctcgt tcaggcactc 1860

ctagaagtag tatccgcgtt ggtgagcatc ttaggcagct cctcgatagg tcaaataaac 1920

tatggtgctt cagcccagta tacacagatg gtgggacaga gcgtcgcgca ggcattggct 1980

taa 1983

<210> 7
<211> 542
<212> PRT
<213> Artificial Sequence

<220>
<223> recombinant DNA of spider silk protein gene ADF3Kai

<400> 7

Met His His His His His His His His His His Ser Ser Gly Ser Ser
1               5                   10                  15

Leu Glu Val Leu Phe Gln Gly Pro Ala Arg Ala Gly Ser Gly Gln Gln
            20                  25                  30

Gly Pro Gly Gln Gln Gly Pro Gly Gln Gln Gly Pro Gly Gln Gln Gly
        35                  40                  45

Pro Tyr Gly Pro Gly Ala Ser Ala Ala Ala Ala Ala Ala Gly Gly Tyr
    50                  55                  60

Gly Pro Gly Ser Gly Gln Gln Gly Pro Ser Gln Gln Gly Pro Gly Gln
65                  70                  75                  80

Gln Gly Pro Gly Gly Gln Gly Pro Tyr Gly Pro Gly Ala Ser Ala Ala
                85                  90                  95

Ala Ala Ala Ala Gly Gly Tyr Gly Pro Gly Ser Gly Gln Gln Gly Pro
            100                 105                 110

Gly Gly Gln Gly Pro Tyr Gly Pro Gly Ser Ser Ala Ala Ala Ala Ala
        115                 120                 125

Ala Gly Gly Asn Gly Pro Gly Ser Gly Gln Gln Gly Ala Gly Gln Gln
    130                 135                 140

Gly Pro Gly Gln Gln Gly Pro Gly Ala Ser Ala Ala Ala Ala Ala Ala
145                 150                 155                 160

Gly Gly Tyr Gly Pro Gly Ser Gly Gln Gln Gly Pro Gly Gln Gln Gly
                165                 170                 175

Pro Gly Gly Gln Gly Pro Tyr Gly Pro Gly Ala Ser Ala Ala Ala Ala
            180                 185                 190

Ala Ala Gly Gly Tyr Gly Pro Gly Ser Gly Gln Gly Pro Gly Gln Gln
        195                 200                 205

Gly Pro Gly Gly Gln Gly Pro Tyr Gly Pro Gly Ala Ser Ala Ala Ala
    210                 215                 220

Ala Ala Ala Gly Gly Tyr Gly Pro Gly Ser Gly Gln Gln Gly Pro Gly
225                 230                 235                 240

Gln Gln Gly Pro Gly Gln Gln Gly Pro Gly Gly Gln Gly Pro Tyr Gly
                245                 250                 255

Pro Gly Ala Ser Ala Ala Ala Ala Ala Ala Gly Gly Tyr Gly Pro Gly
            260                 265                 270

Tyr Gly Gln Gln Gly Pro Gly Gln Gln Gly Pro Gly Gly Gln Gly Pro
        275                 280                 285

Tyr Gly Pro Gly Ala Ser Ala Ala Ser Ala Ala Ser Gly Gly Tyr Gly
    290                 295                 300

Pro Gly Ser Gly Gln Gln Gly Pro Gly Gln Gln Gly Pro Gly Gly Gln
305                 310                 315                 320

Gly Pro Tyr Gly Pro Gly Ala Ser Ala Ala Ala Ala Ala Ala Gly Gly
                325                 330                 335

Tyr Gly Pro Gly Ser Gly Gln Gln Gly Pro Gly Gln Gln Gly Pro Gly
            340                 345                 350

Gln Gln Gly Pro Gly Gln Gln Gly Pro Gly Gly Gln Gly Pro Tyr Gly
        355                 360                 365

Pro Gly Ala Ser Ala Ala Ala Ala Ala Ala Gly Gly Tyr Gly Pro Gly
    370                 375                 380

Ser Gly Gln Gln Gly Pro Gly Gln Gln Gly Pro Gly Gln Gln Gly Pro
385                 390                 395                 400

Gly Gln Gln Gly Pro Gly Gln Gln Gly Pro Gly Gln Gln Gly Pro Gly
                405                 410                 415

Gln Gln Gly Pro Gly Gln Gln Gly Pro Gly Gln Gln Gly Pro Gly Gly
            420                 425                 430

Gln Gly Ala Tyr Gly Pro Gly Ala Ser Ala Ala Ala Gly Ala Ala Gly
        435                 440                 445

Gly Tyr Gly Pro Gly Ser Gly Gln Gln Gly Pro Gly Gln Gln Gly Pro
    450                 455                 460

Gly Gln Gln Gly Pro Gly Gln Gln Gly Pro Gly Gln Gln Gly Pro Gly
465                 470                 475                 480

Gln Gln Gly Pro Gly Gln Gln Gly Pro Gly Gln Gln Gly Pro Tyr Gly
                485                 490                 495

Pro Gly Ala Ser Ala Ala Ala Ala Ala Ala Gly Gly Tyr Gly Pro Gly
            500                 505                 510

Ser Gly Gln Gln Gly Pro Gly Gln Gln Gly Pro Gly Gln Gln Gly Pro
        515                 520                 525

Gly Gly Gln Gly Pro Tyr Gly Pro Gly Ala Ala Ser Ala Ala
    530                 535                 540

<210>  8
<211>  434
<212>  PRT
<213>  Artificial

<220>
<223>  Recombinang spider silk protein ADF4Kai

<400>  8
   Met His His His His His His His His His His Ser Ser Gly Ser Ser
   1               5                   10                  15
   Leu Glu Val Leu Phe Gln Gly Pro Ala Gly Ser Ser Ala Ala Ala Ala
               20                  25                  30
   Ala Ala Ala Ser Gly Ser Gly Gly Tyr Gly Pro Glu Asn Gln Gly Pro
           35                  40                  45
   Ser Gly Pro Val Ala Tyr Gly Pro Gly Gly Pro Val Ser Ser Ala Ala
       50                  55                  60
   Ala Ala Ala Ala Ala Gly Ser Gly Pro Gly Gly Tyr Gly Pro Glu Asn
   65                  70                  75                  80
   Gln Gly Pro Ser Gly Pro Gly Gly Tyr Gly Pro Gly Gly Ser Gly Ser
                   85                  90                  95
   Ser Ala Ala Ala Ala Ala Ala Ala Ala Ser Gly Pro Gly Gly Tyr Gly
               100                 105                 110
   Pro Gly Ser Gln Gly Pro Ser Gly Pro Gly Gly Ser Gly Gly Tyr Gly
           115                 120                 125
   Pro Gly Ser Gln Gly Ala Ser Gly Pro Gly Gly Pro Gly Ala Ser Ala
       130                 135                 140
   Ala Ala Ala Ala Ala Ala Ala Ala Ala Ser Gly Pro Gly Gly Tyr Gly
   145                 150                 155                 160
   Pro Gly Ser Gln Gly Pro Ser Gly Pro Gly Ala Tyr Gly Pro Gly Gly
                   165                 170                 175
   Pro Gly Ser Ser Ala Ala Ala Ala Ala Ala Ala Ala Ser Gly Pro Gly
               180                 185                 190
   Gly Tyr Gly Pro Gly Ser Gln Gly Pro Ser Gly Pro Gly Val Tyr Gly
           195                 200                 205
   Pro Gly Gly Pro Gly Ser Ser Ala Ala Ala Ala Ala Ala Ala Gly Ser
       210                 215                 220
   Gly Pro Gly Gly Tyr Gly Pro Glu Asn Gln Gly Pro Ser Gly Pro Gly
   225                 230                 235                 240
   Gly Tyr Gly Pro Gly Gly Ser Gly Ser Ser Ala Ala Ala Ala Ala Ala
                   245                 250                 255
   Ala Ala Ser Gly Pro Gly Gly Tyr Gly Pro Gly Ser Gln Gly Pro Ser
               260                 265                 270
   Gly Pro Gly Gly Ser Gly Gly Tyr Gly Pro Gly Ser Gln Gly Gly Ser
           275                 280                 285
   Gly Pro Gly Ala Ser Ala Ala Ala Ala Ala Ala Ala Ala Ser Gly Pro
       290                 295                 300
   Gly Gly Tyr Gly Pro Gly Ser Gln Gly Pro Ser Gly Pro Gly Tyr Gln
   305                 310                 315                 320
   Gly Pro Ser Gly Pro Gly Ala Tyr Gly Pro Ser Pro Ser Ala Ser Ala
                   325                 330                 335
   Ser Val Ala Ala Ser Val Tyr Leu Arg Leu Gln Pro Arg Leu Glu Val
               340                 345                 350
   Ser Ser Ala Val Ser Ser Leu Val Ser Ser Gly Pro Thr Asn Gly Ala
           355                 360                 365
   Ala Val Ser Gly Ala Leu Asn Ser Leu Val Ser Gln Ile Ser Ala Ser
       370                 375                 380
   Asn Pro Gly Leu Ser Gly Cys Asp Ala Leu Val Gln Ala Leu Leu Glu
   385                 390                 395                 400
   Leu Val Ser Ala Leu Val Ala Ile Leu Ser Ser Ala Ser Ile Gly Gln
                   405                 410                 415
   Val Asn Val Ser Ser Val Ser Gln Ser Thr Gln Met Ile Ser Gln Ala
               420                 425                 430
   Leu Ser

<210>  9
<211>  355
<212>  PRT
<213>  Artificial

<220>
<223>  Recombinant spider silk protein MaSp2_N

<400>  9

Met His His His His His His Ser Ser Gly Ser Ser Leu Glu Val Leu
1               5                   10                  15

Phe Gln Gly Pro Ala Arg Ala Gly Pro Gly Gly Tyr Arg Pro Gly Gln
            20                  25                  30

Gln Gly Pro Ser Gly Pro Gly Ser Ala Ala Ala Ala Ala Ala Ala Ala
        35                  40                  45

Ala Gly Pro Gly Gly Tyr Gly Pro Gly Gln Gln Gly Pro Gly Gly Tyr
    50                  55                  60

Gly Pro Gly Gln Gln Gly Pro Ser Gly Ala Gly Ser Ala Ala Ala Ala
65                  70                  75                  80

Ala Ala Ala Gly Pro Gly Gln Gln Gly Leu Gly Gly Tyr Gly Pro Gly
                85                  90                  95

Gln Gln Gly Pro Gly Gly Tyr Gly Pro Gly Gln Gln Gly Pro Gly Gly
            100                 105                 110

Tyr Gly Pro Gly Ser Ala Ser Ala Ala Ala Ala Ala Ala Gly Pro Gly
        115                 120                 125

Gln Gln Gly Pro Gly Gly Tyr Gly Pro Gly Gln Gln Gly Pro Ser Gly
    130                 135                 140

Pro Gly Ser Ala Ser Ala Ala Ala Ala Ala Ala Gly Pro Gly Gly Tyr
145                 150                 155                 160

Gly Pro Gly Gln Gln Gly Pro Gly Gly Tyr Ala Pro Gly Gln Gln Gly
                165                 170                 175

Pro Ser Gly Pro Gly Ser Ala Ala Ala Ala Ala Ala Ala Ala Arg Ala
            180                 185                 190

Gly Pro Gly Gly Tyr Gly Pro Ala Gln Gln Gly Pro Ser Gly Pro Gly
        195                 200                 205

Ile Ala Ala Ser Ala Ala Ser Ala Gly Pro Gly Gly Tyr Gly Pro Ala
    210                 215                 220

Gln Gln Gly Pro Ala Gly Tyr Gly Pro Gly Ser Ala Val Ala Ala Ser
225                 230                 235                 240

Ala Gly Ala Gly Ser Ala Gly Tyr Gly Pro Gly Ser Gln Ala Ser Ala
                245                 250                 255

Ala Ala Ser Arg Leu Ala Ser Pro Asp Ser Gly Ala Arg Val Ala Ser
            260                 265                 270

Ala Val Ser Asn Leu Val Ser Ser Gly Pro Thr Ser Ser Ala Ala Leu
        275                 280                 285

Ser Ser Val Ile Ser Asn Ala Val Ser Gln Ile Gly Ala Ser Asn Pro
    290                 295                 300

Gly Leu Ser Gly Cys Asp Val Leu Ile Gln Ala Leu Leu Glu Ile Val
305                 310                 315                 320

Ser Ala Cys Val Thr Ile Leu Ser Ser Ser Ser Ile Gly Gln Val Asn
                325                 330                 335

Tyr Gly Ala Ala Ser Gln Phe Ala Gln Val Val Gly Gln Ser Val Leu
            340                 345                 350

Ser Ala Phe
        355

<210>  10
<211>  559
<212>  PRT
<213>  Artificial

<220>
<223>  Recombinant spider silk protein Flag_92_short2

<400>  10

Met His His His His His His His His His His Ser Ser Gly Ser Ser
1               5                   10                  15

Leu Glu Val Leu Phe Gln Gly Pro Gly Ala Gly Gly Ser Gly Pro Gly
            20                  25                  30

Gly Ala Gly Pro Gly Gly Val Gly Pro Gly Gly Ser Gly Pro Gly Gly
        35                  40                  45

Val Gly Pro Gly Gly Ser Gly Pro Gly Gly Val Gly Pro Gly Gly Ser
    50                  55                  60

Gly Pro Gly Gly Val Gly Pro Gly Gly Ala Gly Gly Pro Tyr Gly Pro
65                  70                  75                  80

Gly Gly Ser Gly Pro Gly Gly Ala Gly Gly Ala Gly Gly Pro Gly Gly
                85                  90                  95

Ala Tyr Gly Pro Gly Gly Ser Tyr Gly Pro Gly Gly Ser Gly Gly Pro
            100                 105                 110

Gly Gly Ala Gly Gly Pro Tyr Gly Pro Gly Gly Glu Gly Pro Gly Gly
        115                 120                 125

Ala Gly Gly Pro Tyr Gly Pro Gly Gly Ala Gly Gly Pro Tyr Gly Pro
    130                 135                 140

Gly Gly Ala Gly Gly Pro Tyr Gly Pro Gly Gly Glu Gly Gly Pro Tyr
145                 150                 155                 160

Gly Pro Gly Gly Ser Tyr Gly Pro Gly Gly Ala Gly Gly Pro Tyr Gly
                165                 170                 175

Pro Gly Gly Pro Tyr Gly Pro Gly Gly Glu Gly Pro Gly Gly Ala Gly
            180                 185                 190

Gly Pro Tyr Gly Pro Gly Gly Val Gly Pro Gly Gly Gly Gly Pro Gly
        195                 200                 205

Gly Tyr Gly Pro Gly Gly Ala Gly Pro Gly Gly Tyr Gly Pro Gly Gly
    210                 215                 220

Ser Gly Pro Gly Gly Tyr Gly Pro Gly Gly Ser Gly Pro Gly Gly Tyr
225                 230                 235                 240

Gly Pro Gly Gly Ser Gly Pro Gly Gly Tyr Gly Pro Gly Gly Ser Gly
                245                 250                 255

Pro Gly Gly Tyr Gly Pro Gly Gly Ser Gly Pro Gly Gly Ser Gly Pro
            260                 265                 270

Gly Gly Tyr Gly Pro Gly Gly Ser Gly Pro Gly Gly Ser Gly Pro Gly
        275                 280                 285

Gly Tyr Gly Pro Gly Gly Ser Gly Pro Gly Gly Tyr Gly Pro Gly Gly
    290                 295                 300

Ser Gly Pro Gly Gly Ser Gly Pro Gly Gly Tyr Gly Pro Gly Gly Ser
305                 310                 315                 320

Gly Pro Gly Gly Ser Gly Pro Gly Gly Tyr Gly Pro Gly Gly Ser Gly
                325                 330                 335

Pro Gly Gly Phe Gly Pro Gly Gly Phe Gly Pro Gly Gly Ser Gly Pro
            340                 345                 350

Gly Gly Tyr Gly Pro Gly Gly Ser Gly Pro Gly Gly Ala Gly Pro Gly
        355                 360                 365

Gly Val Gly Pro Gly Gly Phe Gly Pro Gly Gly Ala Gly Pro Gly Gly
    370                 375                 380

Ala Gly Pro Gly Gly Ala Gly Pro Gly Gly Ala Gly Pro Gly Gly Ala
385                 390                 395                 400

Gly Pro Gly Gly Ala Gly Pro Gly Gly Ala Gly Pro Gly Gly Ala Gly
                405                 410                 415

Pro Gly Gly Ala Gly Gly Ala Gly Gly Ala Gly Gly Ala Gly Gly Ser
            420                 425                 430

Gly Gly Ala Gly Gly Ser Gly Gly Thr Thr Ile Ile Glu Asp Leu Asp
        435                 440                 445

Ile Thr Ile Asp Gly Ala Asp Gly Pro Ile Thr Ile Ser Glu Glu Leu
    450                 455                 460

Thr Ile Ser Ala Tyr Tyr Pro Ser Ser Arg Val Pro Asp Met Val Asn
465                 470                 475                 480

Gly Ile Met Ser Ala Met Gln Gly Ser Gly Phe Asn Tyr Gln Met Phe
                485                 490                 495

Gly Asn Met Leu Ser Gln Tyr Ser Ser Gly Ser Gly Thr Cys Asn Pro
            500                 505                 510

Asn Asn Val Asn Val Leu Met Asp Ala Leu Leu Ala Ala Leu His Cys
        515                 520                 525

Leu Ser Asn His Gly Ser Ser Ser Phe Ala Pro Ser Pro Thr Pro Ala
    530                 535                 540

Ala Met Ser Ala Tyr Ser Asn Ser Val Gly Arg Met Phe Ala Tyr
545                 550                 555

<210>  11
<211>  1629
<212>  DNA
<213>  Artificial Sequence

<220>
<223>  recombinant spider silk protein gene ADF3Kai-noNR

<400>  11
atgcatcacc atcatcatca tcaccaccac cattcctcgg gctcatcctt ggaagtgtta      60

tttcaaggac cagcacgagc cggttcggga caacaagggc ctggccagca gggcccaggt     120

caacaagggc caggacagca gggtccttat gggcccggcg caagcgcagc agctgcggcc     180

gctggtggct atggtcctgg ctccggtcaa cagggccctt cgcaacaagg tcccgggcag     240

caaggtcctg gtggccaggg tccctacggg ccgggggcga gtgcggcagc agccgctgca     300

ggcggttatg gtccaggaag cggacagcaa ggtccgggag gtcaaggtcc gtatggccca     360

ggctctagcg cggctgccgc tgccgcgggt ggcaacggac cagggagcgg acaacagggc 420

gcgggacaac agggtccagg acagcaaggc ccaggggcgt cggcggctgc agcggcggcc 480

ggaggctatg gacccggctc aggacaacag ggaccgggtc aacaaggacc cggtggccaa 540

ggcccctatg gcccgggcgc cagcgcggcc gcagccgccg cgggcgggta cggccccggt 600

agcggccagg gaccaggtca gcaggggcca ggaggtcagg gcccatacgg tccgggcgca 660

tccgcggcgg cggcagcggc aggtggctac ggtcccggaa gcggccaaca ggggccaggg 720

caacaaggac caggacaaca aggtcctggg ggccaaggac cgtatggacc aggagcatca 780

gctgcagccg cggcagctgg cggttacggt ccaggctacg gccagcaggg tccgggtcag 840

cagggaccgg gaggccaggg gccttatggc cctggcgctt ccgcagccag tgccgcttct 900

ggaggatacg ggccgggaag cggtcagcaa ggccctggcc aacaaggacc tggaggccaa 960

gggccctacg gcccaggagc ctcggcagcc gcagctgccg caggtgggta tgggccaggt 1020

agcgggcaac aagggccggg tcagcaagga ccggggcaac agggacctgg gcagcaagga 1080

cccgggggtc aaggcccgta cggacctggt gcgtctgcag ctgctgctgc ggctggtgga 1140

tatggtccgg gatcggggca gcagggtccc ggtcagcagg gccctggtca gcaagggcca 1200

ggccaacagg gacccggaca acaaggcccg ggtcaacagg gtcctggaca gcaggggccg 1260

ggccaacaag gccctgggca acagggtccg gggggacagg gggcctatgg gcctggcgca 1320

tctgccgccg ctggcgcagc cggtgggtac gggcctgggt caggtcaaca ggggcctggt 1380

caacaaggcc ccgggcaaca gggccccggc cagcaaggtc cagggcagca gggcccggga 1440

cagcaagggc ctggacaaca ggggcccgga cagcagggac cttacgggcc cggtgcgagc 1500

gcagcggccg ccgccgcagg gggatatggc cccggatcgg gccagcaggg accaggccag 1560

caaggacctg gccaacaggg cccggggggt caggggccgt atggtcccgg cgctgcaagt 1620

gctgcataa 1629

<210> 12
<211> 590
<212> PRT
<213> Artificial Sequence

<220>
<223> Met_CRY1_L_A5_giza_QQQ (GizaQ)

<400> 12

Met Gly Pro Gly Gln Gln Gly Pro Tyr Gly Pro Gly Ala Ser Ala Ala
1               5                   10                  15

Ala Ala Ala Gly Gln Asn Gly Pro Gly Ser Gly Gln Gln Gly Pro Gly
            20                  25                  30

Gln Ser Gly Gln Tyr Gly Pro Gly Gln Gln Gly Pro Gly Gln Gln Gly
        35                  40                  45

Pro Gly Ser Ser Ala Ala Ala Ala Ala Gly Pro Gly Gln Tyr Gly Pro
    50                  55                  60

Gly Gln Gln Gly Pro Ser Ala Ser Ala Ala Ala Ala Ala Gly Pro Gly
65                  70                  75                  80

Ser Gly Gln Gln Gly Pro Gly Ala Ser Gly Gln Tyr Gly Pro Gly Gln
                85                  90                  95

Gln Gly Pro Gly Gln Gln Gly Pro Gly Ser Ser Ala Ala Ala Ala Ala
            100                 105                 110

Gly Gln Tyr Gly Ser Gly Pro Gly Gln Gln Gly Pro Tyr Gly Ser Ala
        115                 120                 125

Ala Ala Ala Ala Gly Pro Gly Ser Gly Gln Tyr Gly Gln Gly Pro Tyr
    130                 135                 140

Gly Pro Gly Ala Ser Gly Pro Gly Gln Tyr Gly Pro Gly Gln Gln Gly
145                 150                 155                 160

Pro Ser Ala Ser Ala Ala Ala Ala Ala Gly Ser Gly Gln Gln Gly Pro
                165                 170                 175

Gly Gln Tyr Gly Pro Tyr Ala Ser Ala Ala Ala Ala Ala Gly Gln Tyr
            180                 185                 190

Gly Ser Gly Pro Gly Gln Gln Gly Pro Tyr Gly Pro Gly Gln Ser Gly
        195                 200                 205

Ser Gly Gln Gln Gly Pro Gly Gln Gln Gly Pro Tyr Ala Ser Ala Ala
    210                 215                 220

Ala Ala Ala Gly Pro Gly Gln Gln Gly Pro Tyr Gly Pro Gly Ser Ser
225                 230                 235                 240

Ala Ala Ala Ala Ala Gly Gln Tyr Gly Tyr Gly Pro Gly Gln Gln Gly
                245                 250                 255

Pro Tyr Gly Pro Gly Ala Ser Gly Gln Asn Gly Pro Gly Ser Gly Gln
            260                 265                 270

Tyr Gly Pro Gly Gln Gln Gly Pro Gly Gln Ser Ala Ala Ala Ala Ala
        275                 280                 285

Gly Pro Gly Gln Gln Gly Pro Tyr Gly Pro Gly Ala Ser Ala Ala Ala
    290                 295                 300

Ala Ala Gly Gln Tyr Gly Pro Gly Gln Gln Gly Pro Gly Gln Tyr Gly
305                 310                 315                 320

Pro Gly Ser Ser Gly Pro Gly Gln Gln Gly Pro Tyr Gly Pro Gly Ser
                325                 330                 335

Ser Ala Ala Ala Ala Ala Gly Gln Tyr Gly Pro Gly Gln Gln Gly Pro
            340                 345                 350

Tyr Gly Pro Gly Gln Ser Ala Ala Ala Ala Ala Gly Gln Tyr Gln Gln
        355                 360                 365

Gly Pro Gly Gln Gln Gly Pro Tyr Gly Pro Gly Ala Ser Gly Pro Gly
    370                 375                 380

Gln Gln Gly Pro Tyr Gly Pro Gly Ala Ser Ala Ala Ala Ala Ala Gly
385                 390                 395                 400

Pro Gly Gln Tyr Gly Pro Gly Gln Gln Gly Pro Ser Ala Ser Ala Ala
                405                 410                 415

Ala Ala Ala Gly Gln Tyr Gly Ser Gly Pro Gly Gln Tyr Gly Pro Tyr
            420                 425                 430

Gly Pro Gly Gln Ser Gly Pro Gly Ser Gly Gln Gln Gly Gln Gly Pro
        435                 440                 445

Tyr Gly Pro Gly Ala Ser Ala Ala Ala Ala Ala Gly Gln Tyr Gly Pro
    450                 455                 460

Gly Gln Gln Gly Pro Tyr Gly Pro Gly Gln Ser Ala Ala Ala Ala Ala
465                 470                 475                 480

Gly Pro Gly Ser Gly Gln Tyr Gly Pro Gly Ala Ser Gly Gln Asn Gly
                485                 490                 495

Pro Gly Ser Gly Gln Tyr Gly Pro Gly Gln Gln Gly Pro Gly Gln Ser
            500                 505                 510

Ala Ala Ala Ala Ala Gly Gln Tyr Gln Gln Gly Pro Gly Gln Gln Gly
        515                 520                 525

Pro Tyr Gly Pro Gly Ala Ser Ala Ala Ala Ala Ala Gly Gln Tyr Gly
    530                 535                 540

Ser Gly Pro Gly Gln Gln Gly Pro Tyr Gly Pro Gly Gln Ser Gly Ser
545                 550                 555                 560

Gly Gln Gln Gly Pro Gly Gln Gln Gly Pro Tyr Ala Ser Ala Ala Ala
                565                 570                 575

Ala Ala Gly Pro Gly Ser Gly Gln Gln Gly Pro Gly Ala Ser
            580                 585                 590

<210>  13
<211>  601
<212>  PRT
<213>  Artificial Sequence

<220>
<223>  CRY1_L_A5_giza_QQQ (GizaQ)

<400>  13

Met His His His His His His Ser Ser Gly Ser Ser Gly Pro Gly Gln
1               5                   10                  15

Gln Gly Pro Tyr Gly Pro Gly Ala Ser Ala Ala Ala Ala Ala Gly Gln
            20                  25                  30

Asn Gly Pro Gly Ser Gly Gln Gln Gly Pro Gly Gln Ser Gly Gln Tyr
        35                  40                  45

Gly Pro Gly Gln Gln Gly Pro Gly Gln Gln Gly Pro Gly Ser Ser Ala
    50                  55                  60

Ala Ala Ala Ala Gly Pro Gly Gln Tyr Gly Pro Gly Gln Gln Gly Pro
65                  70                  75                  80

Ser Ala Ser Ala Ala Ala Ala Ala Gly Pro Gly Ser Gly Gln Gln Gly
                85                  90                  95

Pro Gly Ala Ser Gly Gln Tyr Gly Pro Gly Gln Gln Gly Pro Gly Gln
            100                 105                 110

Gln Gly Pro Gly Ser Ser Ala Ala Ala Ala Ala Gly Gln Tyr Gly Ser
        115                 120                 125

Gly Pro Gly Gln Gln Gly Pro Tyr Gly Ser Ala Ala Ala Ala Ala Gly
    130                 135                 140

Pro Gly Ser Gly Gln Tyr Gly Gln Gly Pro Tyr Gly Pro Gly Ala Ser
145                 150                 155                 160

Gly Pro Gly Gln Tyr Gly Pro Gly Gln Gln Gly Pro Ser Ala Ser Ala
                165                 170                 175

Ala Ala Ala Ala Gly Ser Gly Gln Gln Gly Pro Gly Gln Tyr Gly Pro
            180                 185                 190

Tyr Ala Ser Ala Ala Ala Ala Ala Gly Gln Tyr Gly Ser Gly Pro Gly
        195                 200                 205

Gln Gln Gly Pro Tyr Gly Pro Gly Gln Ser Gly Ser Gly Gln Gln Gly
    210                 215                 220

Pro Gly Gln Gln Gly Pro Tyr Ala Ser Ala Ala Ala Ala Ala Gly Pro
225                 230                 235                 240

Gly Gln Gln Gly Pro Tyr Gly Pro Gly Ser Ser Ala Ala Ala Ala Ala
                245                 250                 255

Gly Gln Tyr Gly Tyr Gly Pro Gly Gln Gln Gly Pro Tyr Gly Pro Gly
            260                 265                 270

Ala Ser Gly Gln Asn Gly Pro Gly Ser Gly Gln Tyr Gly Pro Gly Gln
        275                 280                 285

Gln Gly Pro Gly Gln Ser Ala Ala Ala Ala Ala Gly Pro Gly Gln Gln
    290                 295                 300

Gly Pro Tyr Gly Pro Gly Ala Ser Ala Ala Ala Ala Ala Gly Gln Tyr
305                 310                 315                 320

Gly Pro Gly Gln Gln Gly Pro Gly Gln Tyr Gly Pro Gly Ser Ser Gly
                325                 330                 335

Pro Gly Gln Gln Gly Pro Tyr Gly Pro Gly Ser Ser Ala Ala Ala Ala
            340                 345                 350

Ala Gly Gln Tyr Gly Pro Gly Gln Gln Gly Pro Tyr Gly Pro Gly Gln
        355                 360                 365

Ser Ala Ala Ala Ala Ala Gly Gln Tyr Gln Gln Gly Pro Gly Gln Gln
    370                 375                 380

Gly Pro Tyr Gly Pro Gly Ala Ser Gly Pro Gly Gln Gln Gly Pro Tyr
385                 390                 395                 400

Gly Pro Gly Ala Ser Ala Ala Ala Ala Ala Gly Pro Gly Gln Tyr Gly
                405                 410                 415

Pro Gly Gln Gln Gly Pro Ser Ala Ser Ala Ala Ala Ala Ala Gly Gln
            420                 425                 430

Tyr Gly Ser Gly Pro Gly Gln Tyr Gly Pro Tyr Gly Pro Gly Gln Ser
        435                 440                 445

Gly Pro Gly Ser Gly Gln Gln Gly Gln Gly Pro Tyr Gly Pro Gly Ala
    450                 455                 460

Ser Ala Ala Ala Ala Ala Gly Gln Tyr Gly Pro Gly Gln Gln Gly Pro
465                 470                 475                 480

Tyr Gly Pro Gly Gln Ser Ala Ala Ala Ala Ala Gly Pro Gly Ser Gly
                485                 490                 495

Gln Tyr Gly Pro Gly Ala Ser Gly Gln Asn Gly Pro Gly Ser Gly Gln
            500                 505                 510

Tyr Gly Pro Gly Gln Gln Gly Pro Gly Gln Ser Ala Ala Ala Ala Ala
        515                 520                 525

Gly Gln Tyr Gln Gln Gly Pro Gly Gln Gln Gly Pro Tyr Gly Pro Gly
    530                 535                 540

Ala Ser Ala Ala Ala Ala Ala Gly Gln Tyr Gly Ser Gly Pro Gly Gln
545                 550                 555                 560

Gln Gly Pro Tyr Gly Pro Gly Gln Ser Gly Ser Gly Gln Gln Gly Pro
                565                 570                 575

Gly Gln Gln Gly Pro Tyr Ala Ser Ala Ala Ala Ala Ala Gly Pro Gly
            580                 585                 590

Ser Gly Gln Gln Gly Pro Gly Ala Ser
        595                 600

<210>  14
<211>  12
<212>  PRT
<213>  Artificial Sequence

<220>
<223>  HisTag

<400>  14

Met His His His His His His Ser Ser Gly Ser Ser
1               5                   10